# EUROPEAN PATENT APPLICATION

(11) **EP 2 724 717 A1**
(43) Date of publication of application: **30.04.2014**
(21) Application number: 12802026.0
(22) Date of filing: 22.06.2012
(51) Int. Cl.: A61K 9/30, A61K 9/16, A61K 47/48, A61K 49/10

(54) **SURFACE TREATMENT METHOD FOR MAGNETIC PARTICLES, MAGNETIC COMPOSITE PREPARED THEREBY, AND MAGNETIC COMPOSITE FOR LABELING TARGET MATERIALS**

(30) Priority: 23.06.2011 KR 20110060931; 23.06.2011 KR 20110060935; 23.06.2011 KR 20110060936
(71) Applicant: Nanobrick Co., Ltd., Suwon-si, Gyeonggi-do 443-270 (KR)
(72) Inventor: JOO, Jae-Hyun, Hwaseong-si Gyeonggi-do 445-855 (KR); WEE, Sung-Bok, Suwon-si Gyeonggi-do 442-819 (KR); PARK, Youn-Jung, Suwon-si Gyeonggi-do 441-440 (KR); HONG, Sung-Wan, Yongin-si Gyeonggi-do 448-140 (KR); CHANG, Hyong-Ku, Namyangju-si Gyeonggi-do 472-724 (KR)
(74) Representative: Zardi, Marco
(86) International application number: PCT/KR2012/004969
(87) International publication number: WO 2012/177088

(57) **Abstract**

The present invention relates to a surface treatment method for magnetic particles, a magnetic composite prepared thereby, and a magnetic composite for labeling target materials. More specifically, the invention relates to a surface treatment method for magnetic particles and a magnetic composite having excellent dispersibility prepared thereby, wherein the surface treatment method comprises the steps of: performing the acid-treatment of magnetic particles and mixing the acid-treated magnetic particles with a water-soluble solvent in order to form a hydroxyl group (-OH) on the surface of the magnetic particles; and mixing the magnetic particles, in which the hydroxyl group is formed, with a surface treatment agent containing an organic ligand, which can be bonded to the hydroxyl group, so that the organic ligand is treated on the surface of the magnetic particles.

## Description

### FIELD OF THE INVENTION

The present invention relates to a surface treatment method for magnetic particles, a magnetic composite prepared thereby, and a magnetic composite for labeling target materials.

### BACKGROUND

Magnetic particles have been utilized in various fields including diagnosis and treatment of diseases such as cancer for medical purposes, immunoassay, contrast media used for medical photographing, drug delivery system (DDS), genetic engineering such as RNA isolation, biosensors for analyzing biological analytes, waste water purification, catalysts, information storage, display technology for forgery prevention, display technology using photonic crystal characteristics, and the like.

Iron oxides mainly used as a material for magnetic particles have toxicity, less oxidation resistance and non-hydrophilic property, and thus need to be surface-treated with an inorganic or organic compound such as tetraethyl orthosilicate (TEOS) or polyethylene glycol (PEG).

However, the surface treatment of iron oxide particles with TEOS or PEG may weaken the magnetism of the iron oxide particles and severely deteriorate the dispersibility thereof in a fat-soluble solvent, causing agglomeration of the individual iron oxide particles. Moreover, since the surface treatment is conducted under a high pressure and at a high temperature, it is highly risky and unsuitable for mass production.

Meanwhile, researches on new technologies by which various bio-molecules and ligands thereof can be promptly screened at the same time using magnetic particles are gaining popularity. It is because the techniques for labeling and detecting target materials such as bio-molecules by binding magnetic particles thereto do not require a separate high-cost apparatus and may detect an infinitesimal amount of the bio-molecules.

However, in the labeling and detecting technology using magnetic particles, surfaces of the magnetic particles need to be modified so that the particles may be attached to the bio-molecules. According to the conventional techniques for surface treatment of magnetic particles, the particles are treated with inorganic/organic compounds and then functional ligands are introduced thereto in order to modify the toxicity, oxidative property and non-hydrophilic property of the magnetic particles. As such, the conventional techniques for surface treatment of magnetic particles require multi-step surface treatment processes, and thus take a long time for manufacturing and are unsuitable for mass production. For example, the conventional techniques may be configured to coat magnetic particles or magnetic nanoparticles with TEOS or PEG and then bind a ligand such as an amine group to the coated TEOS or PEG.

### SUMMARY OF THE INVENTION

In order to solve the above-mentioned problems, one object of the present invention is to provide a surface treatment method for magnetic particles and a magnetic composite prepared thereby having good dispersibility, wherein no surface treatment with TEOS or PEG is required and the magnetic particles may have good dispersibility even in a fat-soluble solvent while having good magnetic characteristics, and may be mass produced even under normal pressure.

Another object of the present invention is to provide a surface treatment method for magnetic particles, a magnetic composite prepared thereby, and a magnetic composite for labeling target materials, wherein the method is suitable for mass production in that surfaces of the magnetic particles are modified with a carboxyl group or silicon oxide containing an amine group through a simpler treatment process compared to the prior art.

In accordance with a first aspect of the present invention, there is provided a surface treatment method for a magnetic particle, comprising the steps of: (a) treating a magnetic particle with acid and then mixing the acid-treated magnetic particle with a water-soluble solvent to form a hydroxy group (-OH) on a surface of the magnetic particle; and (b) mixing the magnetic particle on which the hydroxy group is formed with a surface treatment agent containing an organic ligand capable of bonding to the hydroxy group to introduce the organic ligand onto the surface of the magnetic particle.

In step (a), the magnetic particle may include at least one of Cr, Ni, Ti, Zr, Fe, Co, Zn, Gd, Ta, Nb, Pt, Au, Mg, Mn, Pd, Sr, Ag, Ba, Cu, W, Mo, Sn, and Pb.

In step (a), the acid may include at least one of hydrochloric acid (HCl), acetic acid (CH₃COOH), sulfuric acid (H₂SO₄), nitric acid (HNO₃), formic acid, citric acid, lactic acid, and amino acid.

In step (b), the surface treatment agent containing the organic ligand may include at least one of a carboxyl group, a hydroxy group, an amine group, a vinyl group, an acrylate group, an alcohol group, a ketone group, an ester group, and an aldehyde group.

In step (b), the surface treatment containing the organic ligand may include at least one of ricinoleic acid, linoleic acid, monostearin, palmitic acid, octadecylamin, trioctylphosphine oxide, oleic acid, stearic acid, polymethylmethacrylate, polystyrene, solbitol monooleate, sorbitan trioleate, myristoleic acid, palmitoleic acid, sapienic acid, arachidonic acid, α-linolenic acid, eicosapentaenoic acid, erucic acid, docosahexaenoic acid, trioctylphosphate, hexadecylamino, fatty acid series, and olefin series.

In accordance with a second aspect of the present invention, there is provided a magnetic composite being prepared by (a) treating a magnetic particle with acid and then mixing the acid-treated magnetic particle with a water-soluble solvent to form a hydroxy group (-OH) on a surface of the magnetic particle; and (b) mixing the magnetic particle on which the hydroxy group is formed with a surface treatment agent containing an organic ligand capable of bonding to the hydroxy group to introduce the organic ligand onto the surface of the magnetic particle.

The magnetic particle may be a cluster resulting from agglomeration of a plurality of magnetic particles. When the cluster is dispersed in a water-soluble or fat-soluble solvent, a steric hindrance effect may be generated among the plurality of magnetic particles by the organic ligand formed on the surfaces of the plurality of magnetic particles.

In accordance with a third aspect of the present invention, there is provided a surface treatment method for a magnetic particle, comprising the steps of: (a) mixing a surface precursor with a solvent to prepare a surface treatment agent, the surface precursor containing silicon substituted with at least one alkoxy group and at least one amine group; (b) treating a magnetic particle with acid and then mixing the acid-treated magnetic particle with a water-soluble solvent to form a hydroxy group (-OH) on a surface of the magnetic particle; and (c) mixing the magnetic particle on which the hydroxy group is formed with the surface treatment agent to introduce silicon oxide containing the amine group onto the surface of the magnetic particle.

In step (a), the amine group may be selected from a group consisting of a monoamine group, a diamine group, a triamine group, an ethylene diamine group, and a diethylene triamine group.

In step (a), the surface precursor may include aminopropyltriethoxy-silane.

In step (a), the solvent may include at least one of water and a hydrophilic solvent.

In step (b), the magnetic particle may include at least one of Cr, Ni, Ti, Zr, Fe, Co, Zn, Gd, Ta, Nb, Pt, Au, Mg, Mn, Pd, Sr, Ag, Ba, Cu, W, Mo, Sn, and Pb.

In step (b), the acid may include at least one of hydrochloric acid (HCl), acetic acid (CH₃COOH), sulfuric acid (H₂SO₄), nitric acid (HNO₃), formic acid, citric acid, lactic acid, and amino acid.

Step (c) may be conducted at a temperature ranging from 25 °C to 90 °C.

Step (c) may be conducted in a combination of a first temperature maintenance state at 25 °C to 35 °C and a second temperature maintenance state at 60 °C to 90 °C.

Here, heating in the first temperature maintenance state, heating in the second temperature maintenance state, and heating again in the first temperature maintenance state may be sequentially conducted.

In accordance with a fourth aspect of the present invention, there is provided a magnetic composite for labeling a target material, the magnetic composite being prepared by (a) mixing a surface precursor with a solvent to prepare a surface treatment agent, the surface precursor containing silicon substituted with at least one alkoxy group and at least one amine group; (b) treating a magnetic particle with acid and then mixing the acid-treated magnetic particle with a water-soluble solvent to form a hydroxy group (-OH) on a surface of the magnetic particle; and (c) mixing the magnetic particle on which the hydroxy group is formed with the surface treatment agent to introduce silicon oxide containing the amine group onto the surface of the magnetic particle.

In accordance with a fifth aspect of the present invention, there is provided a magnetic composite for labeling a target material, the magnetic composite being prepared by (a) mixing a surface precursor with a solvent to prepare a surface treatment agent, the surface precursor containing silicon substituted with at least one alkoxy group and at least one amine group; (b) treating a magnetic particle with acid and then mixing the acid-treated magnetic particle with a water-soluble solvent to form a hydroxy group (-OH) on a surface of the magnetic particle; and (c) mixing the magnetic particle on which the hydroxy group is formed with the surface treatment agent to introduce silicon oxide containing the amine group onto the surface of the magnetic particle, wherein the amine group is directly or indirectly used to label a target material including at least one of deoxyribonucleic acid (DNA), ribonucleic acid (RNA), peptide, protein, antigen, antibody, nucleic acid aptamer, hapten, antigen protein, DNA-binding protein, hormone, tumor-specific marker, and tissue-specific marker.

In accordance with a sixth aspect of the present invention, there is provided a surface treatment method for a magnetic particle, comprising the steps of: (a) mixing a surface precursor with a solvent to prepare a surface treatment agent, the surface precursor containing at least one carboxyl group and at least one other functional group capable of undergoing a dehydration reaction with a hydroxy group (-OH); (b) treating a magnetic particle with acid and then mixing the acid-treated magnetic particle with a water-soluble solvent to form a hydroxy group (-OH) on a surface of the magnetic particle; and (c) mixing the magnetic particle on which the hydroxy group is formed with the surface treatment agent to introduce the carboxyl group onto the surface of the magnetic particle through a dehydration reaction of the other functional group with the hydroxy group.

In step (a), the surface precursor may include hydroxy acid based compounds.

In step (a), the solvent may include at least one of water and a hydrophilic solvent.

In step (b), the magnetic particle may include at least one of Cr, Ni, Ti, Zr, Fe, Co, Zn, Gd, Ta, Nb, Pt, Au, Mg, Mn, Pd, Sr, Ag, Ba, Cu, W, Mo, Sn, and Pb.

In step (b), the acid may include at least one of hydrochloric acid (HCl), acetic acid (CH₃COOH), sulfuric acid (H₂SO₄), nitric acid (HNO₃), formic acid, citric acid, lactic acid, and amino acid.

Step (c) may be conducted at a temperature ranging from 25 °C to 90 °C.

Step (c) may be conducted in a combination of a first temperature maintenance state at 25 °C to 35 °C and a second temperature maintenance state at 60 °C to 90 °C.

Here, heating in the first temperature maintenance state, heating in the second temperature maintenance state, and heating again in the first temperature maintenance state may be sequentially conducted.

In accordance with a seventh aspect of the present invention, there is provided a surface treatment method for a magnetic particle, comprising the steps of: (a) mixing a surface precursor with a solvent to prepare a surface treatment agent, the surface precursor containing at least one first carboxyl group and at least one second carboxyl group; (b) treating a magnetic particle with acid and then mixing the acid-treated magnetic particle with a water-soluble solvent to form a hydroxy group (-OH) on a surface of the magnetic particle; and (c) mixing the magnetic particle on which the hydroxy group is formed with the surface treatment agent to introduce the first carboxyl group onto the surface of the magnetic particle through a dehydration reaction of the second carboxyl group with the hydroxy group without a dehydration reaction of the first carboxyl group with the hydroxy group.

In step (a), the surface precursor may include acrylic acid based compounds.

In step (a), the solvent may include at least one of water and a hydrophilic solvent.

In step (b), the magnetic particle may include at least one of Cr, Ni, Ti, Zr, Fe, Co, Zn, Gd, Ta, Nb, Pt, Au, Mg, Mn, Pd, Sr, Ag, Ba, Cu, W, Mo, Sn, and Pb.

In step (b), the acid may include at least one of hydrochloric acid (HCl), acetic acid (CH₃COOH), sulfuric acid (H₂SO₄), nitric acid (HNO₃), formic acid, citric acid, lactic acid, and amino acid.

Step (c) may be conducted at a temperature ranging from 25 °C to 90 °C.

Step (c) may be conducted in a combination of a first temperature maintenance state at 25 °C to 35 °C and a second temperature maintenance state at 60 °C to 90 °C.

Here, heating in the first temperature maintenance state, heating in the second temperature maintenance state, and heating again in the first temperature maintenance state may be sequentially conducted.

In accordance with an eighth aspect of the present invention, there is provided a magnetic composite being prepared by (a) mixing a surface precursor with a solvent to prepare a surface treatment agent, the surface precursor containing at least one carboxyl group and at least one other functional group capable of undergoing a dehydration reaction with a hydroxy group (-OH); (b) treating a magnetic particle with acid and then mixing the acid-treated magnetic particle with a water-soluble solvent to form a hydroxy group (-OH) on a surface of the magnetic particle; and (c) mixing the magnetic particle on which the hydroxy group is formed with the surface treatment agent to introduce the carboxyl group onto the surface of the magnetic particle through a dehydration reaction of the other functional group with the hydroxy group.

In accordance with a ninth aspect of the present invention, there is provided a magnetic composite being prepared by (a) mixing a surface precursor with a solvent to prepare a surface treatment agent, the surface precursor containing at least one first carboxyl group and at least one second carboxyl group; (b) treating a magnetic particle with acid and then mixing the acid-treated magnetic particle with a water-soluble solvent to form a hydroxy group (-OH) on a surface of the magnetic particle; and (c) mixing the magnetic particle on which the hydroxy group is formed with the surface treatment agent to introduce the first carboxyl group onto the surface of the magnetic particle through a dehydration reaction of the second carboxyl group with the hydroxy group without a dehydration reaction of the first carboxyl group with the hydroxy group.

In accordance with a tenth aspect of the present invention, there is provided a magnetic composite for labeling a target material, the magnetic composite being prepared by (a) mixing a surface precursor with a solvent to prepare a surface treatment agent, the surface precursor containing at least one carboxyl group and at least one other functional group capable of undergoing a dehydration reaction with a hydroxy group (-OH); (b) treating a magnetic particle with acid and then mixing the acid-treated magnetic particle with a water-soluble solvent to form a hydroxy group (-OH) on a surface of the magnetic particle; and (c) mixing the magnetic particle on which the hydroxy group is formed with the surface treatment agent to introduce the carboxyl group onto the surface of the magnetic particle through a dehydration reaction of the other functional group with the hydroxy group, wherein the carboxyl group is directly or indirectly used to label a target material including at least one of DNA, RNA, peptide, protein, antigen, antibody, nucleic acid aptamer, hapten, antigen protein, DNA-binding protein, hormone, tumor-specific marker, and tissue-specific marker.

In accordance with an eleventh aspect of the present invention, there is provided a magnetic composite for labeling a target material, the magnetic composite being prepared by (a) mixing a surface precursor with a solvent to prepare a surface treatment agent, the surface precursor containing at least one first carboxyl group and at least one second carboxyl group; (b) treating a magnetic particle with acid and then mixing the acid-treated magnetic particle with a water-soluble solvent to form a hydroxy group (-OH) on a surface of the magnetic particle; and (c) mixing the magnetic particle on which the hydroxy group is formed with the surface treatment agent to introduce the first carboxyl group onto the surface of the magnetic particle through a dehydration reaction of the second carboxyl group with the hydroxy group without a dehydration reaction of the first carboxyl group with the hydroxy group, wherein the first carboxyl group is directly or indirectly used to label a target material including at least one of DNA, RNA, peptide, protein, antigen, antibody, nucleic acid aptamer, hapten, antigen protein, DNA-binding protein, hormone, tumor-specific marker, and tissue-specific marker.

As set forth above, in the surface treatment method for magnetic particles according to the present invention, the magnetic particles are not surface-treated with tetraethyl orthosilicate (TEOS) or polyethylene glycol (PEG), so that the magnetic particles may be well dispersed even in a fat-soluble solvent due to the steric hindrance effect of the organic ligand while retaining good magnetic characteristics. Further, the surface treatment method for magnetic particles according to the present invention neither requires high-pressure production conditions nor coating with TEOS or PEG, thereby enabling mass production through a simple process.

In addition, the surface treatment method for magnetic particles according to the present invention is suitable for mass production since a carboxyl group or silicon oxide containing an amine group is introduced to the magnetic particles through a simple treatment process. Further, the magnetic composite according to another embodiment of the present invention may be applied to a human body and has good dispersibility. Furthermore, the magnetic composite for labeling target materials according to another embodiment of the present invention may label and detect an infinitesimal amount of DNA, RNA, peptide, protein, antigen, antibody, nucleic acid aptamer, hapten, antigen protein, DNA-binding protein, hormone, tumor-specific marker, and tissue-specific marker.

### BRIEF DESCRIPTION OF THE DRAWAINGS

FIG. 1 is a flowchart illustrating a surface treatment method for magnetic particles according to an embodiment of the present invention.
FIG. 2 is a flowchart illustrating a surface treatment method for magnetic particles according to another/yet another embodiment of the present invention.
FIG. 3 is a schematic diagram illustrating a procedure in which a magnetic composite for labeling target materials according to another embodiment of the present invention labels a target material in a living body.
FIG. 4 is a schematic diagram illustrating a procedure in which a magnetic composite for labeling target materials according to yet another embodiment of the present invention labels a target material in a living body.
FIG. 5 is a schematic diagram illustrating a procedure to detect the labeled target material shown in FIG. 3 or 4.
FIG. 6 is an image obtained by analyzing an iron oxide particle at an initial stage (in black) and an iron oxide particle after step (a-2) (in red) in Example 1, using fourier transform-infrared (FT-IR) spectroscopy.
FIG. 7 is an image obtained by analyzing an iron oxide particle at an initial stage (in black) and an iron oxide particle after step (b) (in red) in Example 1, using FT-IR spectroscopy.
FIG. 8 shows data obtained by measuring the degree of dispersion of the particles as step (a-1) (in black), step (a-2) (in red) and step (b) (in blue) are carried out in Example 1.
FIG. 9 is an image obtained by analyzing an iron oxide particle at an initial stage (in black) and an iron oxide particle after the introduction of an organic ligand (in red) in Example 2, using FT-IR spectroscopy.
FIG. 10 shows data obtained by measuring the degree of dispersion of the particles prepared in Example 2.
FIG. 11 is an image obtained by analyzing an iron oxide particle at an initial stage (in black) and an iron oxide particle after the introduction of an organic ligand (in red) in Example 3, using FT-IR spectroscopy.
FIG. 12 shows data obtained by measuring the degree of dispersion of the particles prepared in Example 3.
FIG. 13 is an image obtained by analyzing an iron oxide particle at an initial stage (in black) and an iron oxide particle after the introduction of an organic ligand (in red) in Example 4, using FT-IR spectroscopy.
FIG. 14 shows data obtained by measuring the degree of dispersion of the particles prepared in Example 4.
FIG. 15 is an image obtained by analyzing an iron oxide particle to which silicon oxide and an amine group are introduced after a hydroxy group has been introduced thereto in Example 5, using FT-IR spectroscopy.
FIG. 16 is an image obtained by analyzing an iron oxide particle to which silicon oxide and an amine group are introduced after a hydroxy group has been introduced thereto in Example 6, using FT-IR spectroscopy.
FIG. 17 is an image obtained by analyzing an iron oxide particle to which silicon oxide and an amine group are introduced after a hydroxy group has been introduced thereto in Example 7, using FT-IR spectroscopy.
FIG. 18 is an image obtained by analyzing an iron oxide particle to which a carboxyl group is introduced after a hydroxy group has been introduced thereto in Example 8, using FT-IR spectroscopy.
FIG. 19 is an image obtained by analyzing an iron oxide particle to which a carboxyl group is introduced after a hydroxy group has been introduced thereto in Example 9, using FT-IR spectroscopy.
FIG. 20 is an image obtained by analyzing an iron oxide particle to which a carboxyl group is introduced after a hydroxy group has been introduced thereto in Example 10, using FT-IR spectroscopy.

### DETAILED DESCRIPTION OF THE PREFERRED EMBODIMENTS

In the following detailed description of the present invention, reference is made to the accompanying drawings that show, by way of illustration, specific embodiments in which the present invention may be practiced. These embodiments are described in sufficient detail to enable those skilled in the art to practice the present invention. However, it should be understood that they are not intended to limit the present invention to the particular forms disclosed, but to cover all the modifications, equivalents, and alternatives falling within the spirit and scope of the invention. While such terms as "first" and "second" may be used to describe various elements, those elements are not to be limited by the terms. The above terms are used only to distinguish one element from another. For example, a first element may be referred to as a second element and *vice versa* without departing from the scope of the present invention.

The terms used herein are not intended to limit the present invention but to describe particular embodiments. The singular forms are intended to include the plural forms as well, unless the context clearly indicates otherwise. It should be understood that the terms such as "include" and "have" herein specify the presence of stated features, figures, steps, operations, elements, or combinations thereof, but do not preclude the possibility of presence or addition of one or more other features, figures, steps, operations, elements, or combinations thereof. Unless defined otherwise, all terms used herein including technical or scientific terms have the same meaning as generally understood by a person of ordinary skill in the art to which the present invention pertains.

It shall be noted that terms such as those defined in commonly used dictionaries should be interpreted as having the meaning consistent with the context of the relevant art, and not as having an abnormally or inordinately formal meaning unless they are explicitly defined herein.

Prior to the description of a surface treatment method for magnetic particles according to the present invention, a preparing method of magnetic particles used therefor will be first described. The preparing method of magnetic particles to be described below is provided to illustrate one example of magnetic particles used in the surface treatment method for magnetic particles according to the present invention, and thus shall not be construed to limit the present invention thereto.

The magnetic particles used in the surface treatment method for magnetic particles according to the present invention may include at least one of Cr, Ni, Ti, Zr, Fe, Co, Zn, Gd, Ta, Nb, Pt, Au, Mg, Mn, Pd, Sr, Ag, Ba, Cu, W, Mo, Sn, and Pb. The magnetic particles may be in an oxidized state. In addition, the magnetic particles may contain different kinds of metals. For example, the magnetic particles may be represented by General Formulas 1 to 4 below.

M General Formula 1

(M is a metal element exhibiting magnetism or an alloy thereof.)

MₐO_{b} General Formula 2

(0<a≤20 and 0<b≤20; M is a metal element exhibiting magnetism or an alloy thereof.)

M_{c}M'_{d} General Formula 3

(0<c≤20 and 0<d≤20; M is a metal element exhibiting magnetism or an alloy thereof; and M' is an element selected from a group consisting of Group 2 elements, transition metal elements, Group 13 elements, Group 14 elements, Group 15 elements, lanthanides, and actinides.)

MₐM'ₑO_{b} General Formula 4

(0<a≤20, 0<e≤20, and 0<b≤20; M is a metal element exhibiting magnetism or an alloy thereof; and M' is an element selected from a group consisting of Group 2 elements, transition metal elements, Group 13 elements, Group 14 elements, Group 15 elements, lanthanides, and actinides.)

General Formulas 1 and 3 represent the magnetic particles composed of a single metal or an alloy thereof, and two or more different kinds of metals, respectively. General Formulas 2 and 4 represent the magnetic particles composed of metal oxides containing a single metal or an alloy thereof, and two or more different kinds of metals, respectively.

In the preparing method of magnetic particles, an amorphous metal gel solution is first prepared by adding and dissolving a magnetic precursor and an anionic ligand in a solvent.

Here, the magnetic precursor may be selected from a group consisting of metal nitrate based compounds, metal sulfate based compounds, metal fluoroacetoacetate based compounds, metal halide (MXₐ, M=Cr, Ni, Ti, Zr, Fe, Co, Zn, Gd, Ta, Nb, Pt, Au, Mg, Mn, Pd, Sr, Ag, Ba, Cu, W, Mo, Sn, Pb, X=F, Cl, Br, I, 0<a≤5) based compounds, metal perchlororate based compounds, metal sulfamate based compounds, metal stearate based compounds, and organometal based compounds, but is not necessarily limited thereto.

The anionic ligand may be selected from a group consisting of cationic ligands such as alkyltrimethyl ammonium halides; neutral ligands such as alkyl acid, trialkyl phosphine, trialkyl phosphine oxide, alkyl amine, alkyl thiol and the like; anionic ligands such as sodium alkyl sulfate, sodium alkyl carboxylate, sodium alkyl phosphate, sodium acetate and the like, but is not necessarily limited thereto.

The solvent may be selected from a group consisting of, as organic solvents, aromatic solvents, heterocylclic solvents, sulfoxide based solvents, amide based solvents, hydrocarbon based solvents, ether based solvents, polymer solvents, ionic liquid solvents, halogen hydrocarbon solvents, alcohol based solvents and water, but is not necessarily limited thereto. In some cases, two or more selected from the anionic ligands may be used together or sequentially.

Here, in addition to a single kind of the magnetic precursor, the magnetic particles may further include a hetero-precursor composed of metal halide (MXₐ, M=Cr, Ni, Ti, Zr, Fe, Co, Zn, Gd, Ta, Nb, Pt, Au, Mg, Mn, Pd, Sr, Ag, Ba, Cu, W, Mo, Sn, Pb, X=F, Cl, Br, I; 0<a≤5) based compounds. Metal (M) of the added hetero-precursor is different from the metal contained in the magnetic precursor. Consequently, a magnetic particle containing different kinds of metals may be prepared. The hetero-precursor is preferably added in a content of 1 to 99 parts by weight per 100 parts by weight of the magnetic precursor, but is not necessarily limited thereto. Since the hetero-precursor is added to the magnetic precursor, the finally obtained particle may have enhanced magnetic characteristics or may be variously transformed to exhibit superparamagnetism, paramagnetism, ferromagnetism, antiferromagnetism, ferrimagnetism, diamagnetism and the like, thereby being adjusted to gain desired magnetic characteristics.

Then, the prepared amorphous metal gel solution is heated to be phase-changed into crystalline magnetic particles. For example, the amorphous metal gel solution may be heated at a temperature of 30 °C to 200 °C to form magnetic particles having a more stable crystal structure. In addition, as the magnetic particles are heated again at a temperature of 100 °C to 350 °C to proceed a reduction reaction, a magnetic cluster in which the magnetic particles agglomerate may be prepared.

As described above, magnetic particles such as magnetite (Fe₃O₄), hematite (α-Fe₂O₃) and maghemite (γ-Fe₂O₃) may be prepared.

Further, the magnetic particles may have an average particle size of 1 to 200nm, and a cluster consisting of a plurality of agglomerated magnetic particles may be prepared rather than individual single magnetic particles.

### A. Surface Treatment for Magnetic Particles and Magnetic Composite Prepared Thereby

Hereinafter, a surface treatment method for magnetic particles according to an embodiment of the present invention will be described.

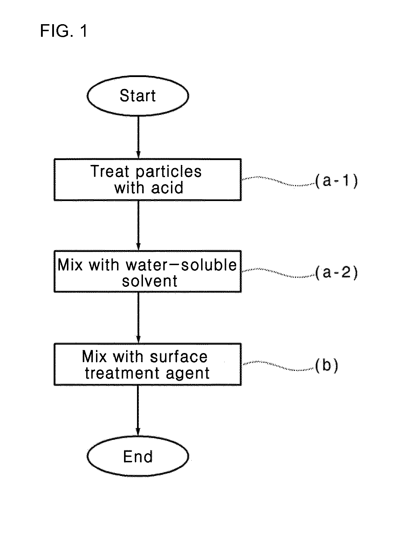

FIG. 1 is a flowchart illustrating a surface treatment method for magnetic particles according to an embodiment of the present invention.

Referring to FIG. 1, in a surface treatment method for magnetic particles according to an embodiment of the present invention, particles are first treated with acid (a-1). Then, the acid-treated particles are mixed with a water-soluble solvent (a-2). Then, the resultant mixture is mixed with a surface treatment agent (b). Hereinafter, the respective steps will be separately described in detail.

In step (a-1) of treating the particles with acid, the acidic material may be variously applied in consideration of acidity according to the components of the magnetic particles. For example, the acid may include at least one of hydrochloric acid (HCl), acetic acid (CH₃COOH), sulfuric acid (H₂SO₄), nitric acid (HNO₃), formic acid, citric acid, lactic acid, and amino acid.

The acid may react with the particles to etch a portion of the surfaces of the particles. Here, the etched surfaces of the particles may be partially charged with positive (+) charges.

In order to allow the above reaction to proceed more effectively, the particles may be mixed with the acid and then stirred using a dispersing unit.

Then, the positively charged particles are mixed with the water soluble solvent (a-2). The water-soluble solvent includes a precursor that may introduce a hydroxy group (-OH) to the positively charged particles. For example, when the water-soluble solvent is water, it may be temporarily bonded to the surfaces of the positively charged particles. As a dehydrogenation reaction of the bonded water occurs, a hydroxy group may be introduced onto the surfaces of the particles. The particles to which the hydroxy group has been introduced may have enhanced dispersibility compared to the initial particles prior to the acid treatment.

Then, an organic ligand is bonded to the hydroxy group of the particle (b). For example, the hydroxy group-introduced particles may be mixed with a material containing the organic ligand and then stirred using a dispersing unit. The organic ligand may include a long alkyl chain and a carboxyl group, an amine group, a vinyl group, or a phenyl group at an end of the alkyl chain. As the carboxyl group and the hydroxy group on the surfaces of the particles are bonded to each other, a long alkyl chain may be formed on the surfaces of the particles.

Here, examples of the material containing an organic ligand may include at least one of ricinoleic acid, linoleic acid, monostearin, palmitic acid, octadecylamin, trioctylphosphine oxide, oleic acid, stearic acid, polymethylmethacrylate, polystyrene, sorbitol monooleate, sorbitan trioleate, myristoleic acid, palmitoleic acid, sapienic acid, arachidonic acid, α-linolenic acid, eicosapentaenoic acid, erucic acid, docosahexaenoic acid, trioctylphosphate, hexadecylamino, fatty acids, and olefins.

When the cluster formed from the agglomeration of a plurality of magnetic particles prepared by the above surface treatment method is dispersed in a water-soluble or fat-soluble solvent, a steric hindrance effect may be generated among the plurality of magnetic particles due to the organic ligand formed on the surfaces of the plurality of magnetic particles.

For example, when the plurality of prepared magnetic particles are exposed to an external strong magnetic field in the solvent, the individual magnetic particles may be easily dispersed in the solvent due to the repulsive force resulting from the steric hindrance effect caused by the alkyl chain in response to the mutual magnetic attraction among the magnetic particles. In addition, the repulsive force among the magnetic particles may be adjusted by treating the end of the alkyl chain with molecules having polarization.

These electrostatic, magnetic and steric hindrance effects may be used to change the intervals between the magnetic particles, thereby exhibiting the structural colors of photo-crystals, which may be applied in the fields of display. In addition, since the prepared magnetic particles are dispersible in a fat-soluble solvent, they may be encapsulated by a coacervation method using oil-in-water (O/W) emulsification. Therefore, the encapsulated magnetic particles may be formed in a desired pattern using a screen printing method, and may be coated on a transparent film and applied as a functional film.

In the surface treatment method for magnetic particles according to the present invention, the magnetic particles are not surface-treated with tetraethyl orthosilicate (TEOS) or polyethylene glycol (PEG), so that the magnetic particles are well dispersed even in a fat-soluble solvent due to the steric hindrance effect while retaining good magnetic characteristics. Further, the method neither requires high-pressure production conditions nor coating with TEOS or PEG, thereby enabling mass production through a simple process.

### B-1. Surface Treatment for Magnetic Particles and Magnetic Composite Prepared Thereby

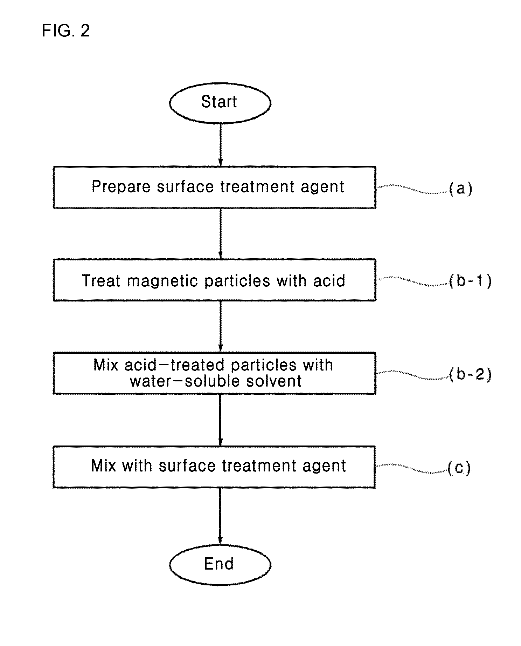

FIG. 2 is a flowchart illustrating a surface treatment method for magnetic particles according to another embodiment of the present invention.

Referring to FIG. 2, in a surface treatment method for magnetic particles according to another embodiment of the present invention, a surface treatment agent is prepared by mixing a surface precursor and a solvent (a). Then, magnetic particles are mixed with the surface treatment agent (b) and (c). Hereinafter, the respective steps will be separately described in detail.

In step (a) of preparing the surface treatment agent by mixing the surface precursor and the solvent, the surface precursor includes silicon substituted with at least one alkoxy group and at least one amine group. The alkoxy group may be bonded with the silicon to have a structure of siloxane (Si-O-R).

For example, the surface precursor may be represented by the following chemical formula:

Rₙ-Si-(OR')₄₋ₙ

wherein R is selected from a group consisting of an amine group, a diamine group, a triamine group, an acid amide group, and aminoxy group, or R is hydrocarbon having a substituent selected from a group consisting of an amine group, a diamine group, a triamine group, an acid amide group, and aminoxy group;
n is an integer of 1 to 3; and
R' is a monovalent alkyl group of 1 to 500 carbon atoms.

For example, the surface precursor may include aminopropyltriethoxy-silane (APS).

The solvent may be variously applied depending on the selection of the surface precursor. For example, the solvent may include water and a hydrophilic solvent. The solvent and the surface precursor may be mixed and then stirred to prepare the surface treatment agent.

Then, the magnetic particles are treated with acid (b-1). Here, the magnetic particles may include at least one of Cr, Ni, Ti, Zr, Fe, Co, Zn, Gd, Ta, Nb, Pt, Au, Mg, Mn, Pd, Sr, Ag, Ba, Cu, W, Mo, Sn, and Pb. In addition, the magnetic particles may contain different kinds of metals or oxidized metals. For example, the magnetic particles may be represented by General Formulas 1 to 4 below.

M General Formula 1

(M is a metal element exhibiting magnetism or an alloy thereof.)

MₐO_{b} General Formula 2

(0<a≤20 and 0<b≤20; M is a metal element exhibiting magnetism or an alloy thereof.)

M_{c}M'_{d} General Formula 3

(0<c≤20 and 0<d≤20; M is a metal element exhibiting magnetism or an alloy thereof; and M' is an element selected from a group consisting of Group 2 elements, transition metal elements, Group 13 elements, Group 14 elements, Group 15 elements, lanthanides, and actinides.)

MₐM'ₑO_{b} General Formula 4

(0<a≤20, 0<e≤20, and 0<b≤20; M is a metal element exhibiting magnetism or an alloy thereof; and M' is an element selected from a group consisting of Group 2 elements, transition metal elements, Group 13 elements, Group 14 elements, Group 15 elements, lanthanides, and actinides.)

General Formulas 1 and 3 represent the magnetic particles composed of a single metal or an alloy thereof, and two or more different kinds of metals, respectively. General Formulas 2 and 4 represent the magnetic particles composed of metal oxides containing a single metal or an alloy thereof, and two or more different kinds of metals, respectively.

The magnetic particles represented by the above general formulas may include magnetite (Fe₃O₄), hematite (α-Fe₂O₃) and maghemite (γ-Fe₂O₃).

The acid may be variously selected in consideration of acidity according to the components of the magnetic particles. For example, the acid may include at least one of hydrochloric acid (HCl), acetic acid (CH₃COOH), sulfuric acid (H₂SO₄), nitric acid (HNO₃), formic acid, citric acid, lactic acid, and amino acid.

The acid may etch a portion of the surfaces of the magnetic particles. Here, the etched surfaces of the magnetic particles may be partially charged with positive (+) charges.

In order to allow the above reaction to proceed more effectively, the magnetic particles may be mixed with the acid and then stirred using a dispersing unit.

Then, the positively charged particles are mixed with the water soluble solvent (b-2). The water-soluble solvent may include a precursor that may introduce a hydroxy group (-OH) to the positively charged particles. For example, when the water-soluble solvent is water, it may be temporarily bonded to the surfaces of the positively charged particles. As a dehydrogenation reaction of the bonded water occurs, a hydroxy group may be introduced to the surfaces of the magnetic particles. The magnetic particles to which the hydroxy group has been introduced may have enhanced dispersibility compared to the initial particles prior to the acid treatment. The magnetic particles prepared in step (b-2) may be a composite in which the magnetic particles are dispersed in the water-soluble solvent, or powder type magnetic particles that have been treated once more.

Then, the surface treatment agent prepared in step (a) and the hydroxy group-introduced magnetic particles are mixed (c). Here, the mixing of the surface treatment agent and the magnetic particles may be conducted at a temperature ranging from 25 °C to 90 °C. When the surface treatment agent and the magnetic particles are mixed at a temperature of 90 °C or higher, the solvent evaporates and the magnetic particles agglomerate with each other, resulting in the lower degree of dispersion. When the surface treatment agent and the magnetic particles are mixed at a temperature of 25 °C or lower, the degree in which an amine group is introduced to the magnetic particles is lowered.

More preferably, step (c) may be conducted by appropriately combining a first temperature maintenance state at 25 °C to 35 °C and a second temperature maintenance state at 60 °C to 90 °C. When the temperature maintenance states are appropriately combined as described above, the degree of dispersion of the magnetic particles and the efficiency of substitution of the amine group may be improved.

For example, heating in the first temperature maintenance state at 25 °C to 35 °C may be first conducted, then heating in the second temperature maintenance state at 60 °C to 90 °C may be conducted, and then heating again in the first temperature maintenance state at 25 °C to 35 °C may be conducted. In this case, the duration of the first temperature maintenance state is preferably longer than that of the second temperature maintenance state.

Alternatively, for example, heating in the second temperature maintenance state at 60 °C to 90 °C may be first conducted, and then heating in the first temperature maintenance state at 25 °C to 35 °C may be conducted. When the temperature maintenance states are combined as described above, the degree of dispersion of the magnetic particles and the efficiency of substitution of the amine group may be improved. In this case, the duration of the second temperature maintenance state is preferably longer than that of the first temperature maintenance state.

In addition, when a mixture of water and 2-aminoethyl)-3-aminopropyl trimethyl-silane is used as the surface treatment agent, the heating in the first temperature maintenance state at 25 °C to 35 °C and the heating in the second temperature maintenance state at 60 °C to 90 °C may be sequentially conducted. Here, even when the first temperature maintenance state is longer than the second temperature maintenance state, desirable results may be obtained.

As described above, in some cases, the first temperature maintenance state at 25 °C to 35 °C and the second temperature maintenance state at 60 °C to 90 °C are appropriately combined so that the degree of dispersion of the particles and the efficiency of substitution of the amine group may be improved.

A surface-treated magnetic composite can be obtained according to the above-described method. The magnetic composite may include a magnetic particle at the center thereof, silicon oxide introduced onto a surface of the magnetic particle, and an amine group introduced onto a surface of the silicon oxide. That is, according to another embodiment of the present invention, silicon oxide and a ligand such as an amine group may be bonded to the magnetic particle through a single step without separately coating the magnetic particle with PEG or TEOS. Thereby, the surface treatment for magnetic particles may be performed in large amounts.

### B-2. Magnetic Composite for Labeling Target Materials and Detection of Target Materials

As described below, the above magnetic composite may be used for labeling and detecting biomolecules as target materials.

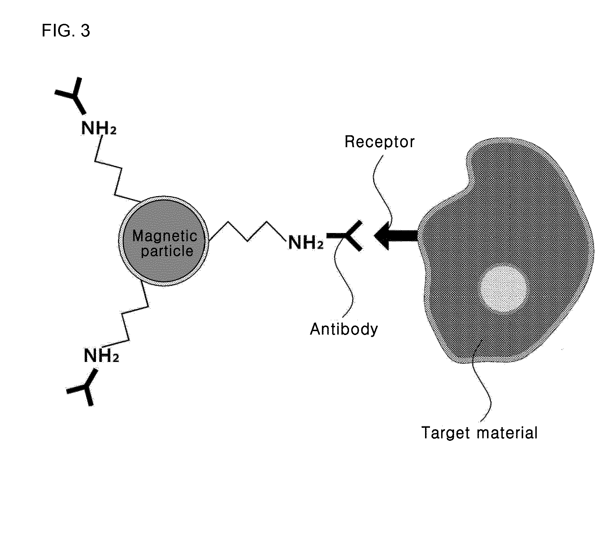

FIG. 3 is a schematic diagram illustrating a procedure in which a magnetic composite for labeling target materials according to another embodiment of the present invention labels a target material in a living body.

Referring to FIG. 3, the amine group, which is a functional ligand of the magnetic composite, may be bonded to an antibody capable of recognizing the target material. The antibody may be specifically bonded to a receptor formed on the cytoplasm of the target material. Here, the target material may include DNA, RNA, peptide, protein, antigen, antibody, nucleic acid aptamer, hapten, antigen protein, DNA-binding protein, hormone, tumor-specific marker, and tissue-specific marker. Alternatively, the target material may include zearalenone, aflatoxin, ochratoxine, patulin, fumonisin, and deoxynivalenol, which are mycotoxins. That is, the amine group of the present magnetic composite is indirectly used to detect a target material by using an antibody bonded to the amine group. Meanwhile, the amine group of the magnetic composite may be directly bonded with the aforementioned target materials through covalent bonding so that it may be used to directly detect the target materials.

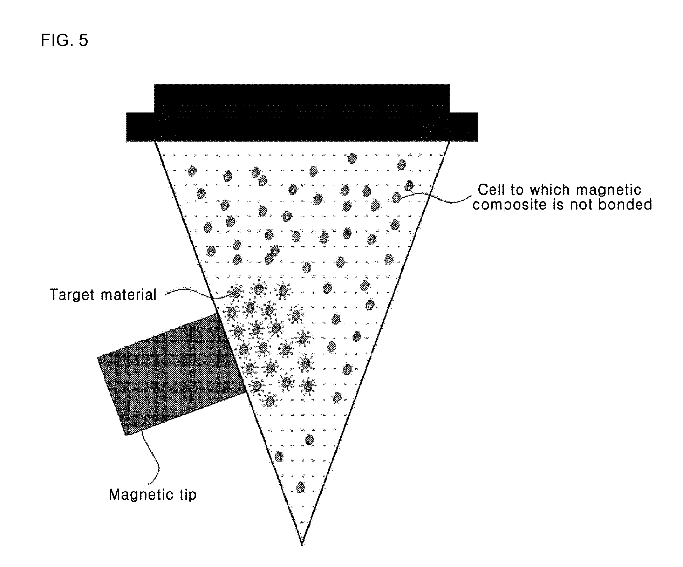

FIG. 5 is a schematic diagram illustrating a procedure to detect the labeled target material shown in FIG. 3.

Referring to FIG. 5, a magnetic tip is allowed to approach a sample in which magnetic composites bonded with target materials and biomolecules not bonded with target materials are irregularly mixed. The target materials bonded with the magnetic composites are concentrated near the magnetic tip due to the magnetism of the magnetic composites. Here, the magnetization value of the concentrated magnetic composites may be measured to detect the content of the target materials.

Here, the magnetic composite or the magnetic particle positioned at the center thereof may have a magnetization value ranging from 20 to 90 emu/g.

In addition, the size of the magnetic composite or the magnetic particle may be variously selected in consideration of the kind and size of target material. For example, if the target material is mycotoxin, the magnetic composite or the magnetic particle may have a size ranging from 10 nm to 300 nm.

As described above, the surface treatment method for magnetic particles according to the present invention is suitable for mass production and has high stability in that the introduction of silicon oxide to surfaces of magnetic particles and the introduction of functional ligands are conducted through a single treatment process.

As the surface treatment agent and the magnetic particles are mixed at a temperature of 25 °C to 90 °C, the surface introduction of an amine group is conducted while the dispersibility among magnetic particles remains high, thereby improving the preparing efficiency.

Further, the magnetic composite according to the present invention has advantages of applicability to a human body and good dispersibility.

Furthermore, the magnetic composite for labeling target materials according to the present invention has an advantage of detecting an infinitesimal amount of DNA, RNA, peptide, protein, and antibody.

### C-1. Surface Treatment for Magnetic Particles and Magnetic Composite Prepared Thereby

FIG. 2 is a flowchart illustrating a surface treatment method for magnetic particles according to yet another embodiment of the present invention.

Referring to FIG. 2, in a surface treatment method for magnetic particles according to yet another embodiment of the present invention, a surface treatment agent is first prepared by mixing a surface precursor and a solvent (a). Then, magnetic particles are mixed with the surface treatment agent (b) and (c). Hereinafter, the respective steps will be separately described in detail.

According to a first case of yet another embodiment of the present invention, in step (a) of preparing the surface treatment agent by mixing the surface precursor and the solvent, the surface precursor includes at least one carboxyl group and at least one other functional group capable of undergoing a dehydration reaction with a hydroxy group (-OH).

The other functional group reacts with a compound having a hydroxy group to separate water. For example, the functional group includes an alkoxy group, a hydroxy group, an amine group, a vinyl group, an acrylate group, an alcohol group, a ketone group, an ester group, and an aldehyde group.

The surface precursor according to the present embodiment may include hydroxy acid based compounds. For example, the precursor includes glycolic acid, lactic acid, malonic acid, malic acid, tartronic acid, glyceric acid, acetic acid, and citric acid.

According to a second case of yet another embodiment of the present invention, in step (a) of preparing the surface treatment agent by mixing the surface precursor and the solvent, the surface precursor may include at least one first carboxyl group and at least one second carboxyl group. As will be described below, the first carboxyl group does not take part in a dehydration reaction in step (c) but only the second carboxyl group takes part in the reaction.

The surface precursor according to the present embodiment includes acrylic acid based compounds. For example, the surface precursor includes methacrylic acid, polyacrylic acid and the like.

The solvent may be variously applied depending on the selection of the surface precursor. For example, the solvent may include at least one of water and a hydrophilic solvent. The solvent and the surface precursor may be mixed and then stirred to prepare the surface treatment agent.

Then, the magnetic particles are treated with acid (b-1). Here, the magnetic particles may include at least one of Cr, Ni, Ti, Zr, Fe, Co, Zn, Gd, Ta, Nb, Pt, Au, Mg, Mn, Pd, Sr, Ag, Ba, Cu, W, Mo, Sn, and Pb. In addition, the magnetic particles may contain different kinds of metals or oxidized metals. For example, the magnetic particles may be expressed by General Formulas 1 to 4 below.

M General Formula 1

(M is a metal element exhibiting magnetism or an alloy thereof.)

MₐO_{b} General Formula 2

(0<a≤20 and 0<b≤20; M is a metal element exhibiting magnetism or an alloy thereof.)

M_{c}M'_{d} General Formula 3

(0<c≤20 and 0<d≤20; M is a metal element exhibiting magnetism or an alloy thereof; and M' is an element selected from a group consisting of Group 2 elements, transition metal elements, Group 13 elements, Group 14 elements, Group 15 elements, lanthanides, and actinides.)

MₐM'ₑO_{b} General Formula 4

(0<a≤20, 0<e≤20, and 0<b≤20; M is a metal element exhibiting magnetism or an alloy thereof; and M' is an element selected from a group consisting of Group 2 elements, transition metal elements, Group 13 elements, Group 14 elements, Group 15 elements, lanthanides, and actinides.)

General Formulas 1 and 3 represent the magnetic particles composed of a single metal or an alloy thereof, and two or more different kinds of metals, respectively. General Formulas 2 and 4 represent the magnetic particles composed of metal oxides containing a single metal or an alloy thereof, and two or more different kinds of metals, respectively.

The magnetic particles represented by the above general formulas may include magnetite (Fe₃O₄), hematite (α-Fe₂O₃) and maghemite (γ-Fe₂O₃).

The acid may be variously selected in consideration of acidity according to the components of the magnetic particles. For example, the acid may include at least one of hydrochloric acid (HCl), acetic acid (CH₃COOH), sulfuric acid (H₂SO₄), nitric acid (HNO₃), formic acid, citric acid, lactic acid, and amino acid.

The acid may etch a portion of the surfaces of the magnetic particles. Here, the etched surfaces of the magnetic particles may be partially charged with positive (+) charges.

In order to allow the above reaction to proceed more effectively, the magnetic particles may be mixed with the acid and then stirred using a dispersing unit.

Then, the positively charged particles are mixed with the water soluble solvent (b-2). The water-soluble solvent may include a precursor that may introduce a hydroxy group (-OH) to the positively charged particles. For example, when the water-soluble solvent is water, it may be temporarily bonded to the surfaces of the positively charged particles. As a dehydrogenation reaction of the bonded water occurs, a hydroxy group may be introduced to the surfaces of the magnetic particles. The magnetic particles to which the hydroxy group has been introduced may have enhanced dispersibility compared to the initial particles prior to the acid treatment.

Then, the surface treatment agent prepared in step (a) and the hydroxy group-introduced magnetic particles are mixed (c). Here, the mixing of the surface treatment agent and the magnetic particles may be conducted at a temperature ranging from 25 °C to 90 °C. When the surface treatment agent and the magnetic particles are mixed at a temperature of 90 °C or higher, the solvent evaporates and the magnetic particles agglomerate with each other, resulting in the lower degree of dispersion. When the surface treatment agent and the magnetic particles are mixed at a temperature of 25 °C or lower, the degree in which the carboxyl group is introduced to the magnetic particles is lowered.

More preferably, step (c) may be conducted by appropriately combining a first temperature maintenance state at 25 °C to 35 °C and a second temperature maintenance state at 60 °C to 90 °C. When the temperature maintenance states are appropriately combined as described above, the degree of dispersion of the particles and the efficiency of substitution of the carboxyl group may be improved.

For example, heating in the first temperature maintenance state at 25 °C to 35 °C may be first conducted, then heating in the second temperature maintenance state at 60 °C to 90 °C may be conducted, and then heating again in the first temperature maintenance state at 25 °C to 35 °C may be conducted. In this case, the duration of the first temperature maintenance state is preferably longer than that of the second temperature maintenance state.

Alternatively, for example, heating in the second temperature maintenance state at 60 °C to 90 °C may be first conducted, and then heating in the first temperature maintenance state at 25 °C to 35 °C may be conducted. When the temperature maintenance states are combined as described above, the degree of dispersion of the magnetic particles and the efficiency of substitution of the carboxyl group may be improved. In this case, the duration of the second temperature maintenance state is preferably longer than that of the first temperature maintenance state.

Through step (c), in the first case of the embodiment, that is, when the surface precursor includes at least one carboxyl group and at least one other functional group capable of undergoing a dehydration reaction with a hydroxy group, the other functional group undergoes a dehydration reaction with the hydroxy group to allow the carboxyl group to be introduced or attached to the surfaces of the magnetic particles.

Meanwhile, through step (c), in the second case of the embodiment, that is, when the surface precursor includes at least one first carboxyl group and at least one second carboxyl group, the first carboxyl group does not undergo a dehydration reaction with the hydroxy group but the second carboxyl group undergoes a dehydration reaction with the hydroxy group to allow the first carboxyl group to be introduced or attached to the surfaces of the magnetic particles.

According to the above-described method, a surface treated magnetic composite may be obtained. The magnetic composite may include a magnetic particle at the center thereof and a carboxyl group introduced onto a surface of the magnetic particle.

That is, according to an embodiment of the present invention, a ligand such as a carboxyl group may be simply bonded to the magnetic particle without separately coating the magnetic particle with PEG or TEOS. Thereby, the surface treatment for magnetic particles may be performed in large amounts.

### C-2. Magnetic Composite for Labeling Target Materials and Detection of Target Materials

As described below, the above magnetic composite may be used for labeling and detecting biomolecules as target materials.

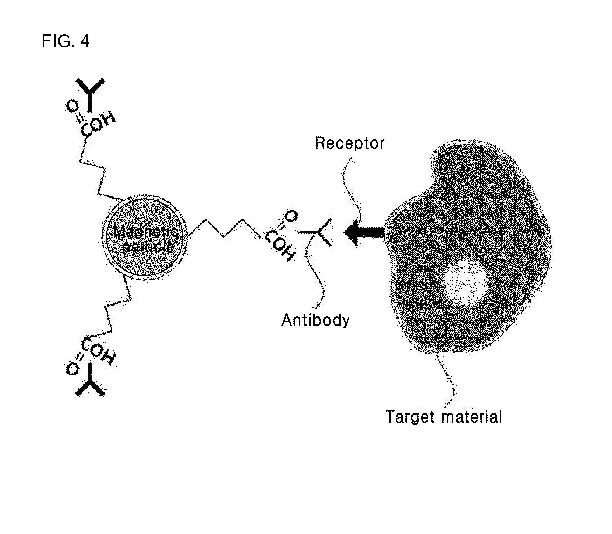

FIG. 4 is a schematic diagram illustrating a procedure in which a magnetic composite for labeling target materials according to yet another embodiment of the present invention labels a target material in a living body.

Referring to FIG. 4, the carboxyl group, which is a functional ligand of the magnetic composite, may be bonded to an antibody capable of recognizing the target material. The antibody may be specifically bonded to a receptor formed on the cytoplasm of the target material. Here, the target material may include DNA, RNA, peptide, protein, antigen, antibody, nucleic acid aptamer, hapten, antigen protein, DNA-binding protein, hormone, tumor-specific marker, and tissue-specific marker. Alternatively, the target material may include zearalenone, aflatoxin, ochratoxine, patulin, fumonisin, and deoxynivalenol, which are mycotoxins. That is, the carboxyl group of the present magnetic composite is indirectly used to detect the target material by using an antibody bonded to the carboxyl group. Meanwhile, the carboxyl group of the magnetic composite may be directly bonded with the aforementioned target materials through covalent bonding so that it may be used to directly detect the target materials.

FIG. 5 is a schematic diagram illustrating a procedure to detect the labeled target material shown in FIG. 4.

Referring to FIG. 5, a magnetic tip is allowed to approach a sample in which magnetic composites bonded with target materials and biomolecules not bonded with target materials are irregularly mixed. The target materials bonded with the magnetic composites are concentrated near the magnetic tip due to magnetism of the magnetic composites. Here, the magnetization value of the concentrated magnetic composites may be measured to detect the content of the target materials. A Giant Magneto Resistance (GMR) sensor may be used in this measurement of detection amount.

Here, the magnetic composite or the magnetic particle positioned at the center thereof may have a magnetization value ranging from 20 to 90 emu/g.

In addition, the size of the magnetic composite or the magnetic particle may be variously selected in consideration of the kind and size of target material. For example, if the target material is mycotoxin, the magnetic composite or the magnetic particle may have a size ranging from 10 nm to 300 nm.

Hereinafter, preferred examples will be set forth to facilitate understanding of the present invention. However, the following examples are merely provided to make it easier to understand the present invention, and the scope of the present invention is not limited by the following examples.

### Example 1

### 1. Preparation of Magnetic Iron Oxide Particles

50 g of iron chloride hydrate as a magnetic precursor, 50 g of sodium hydroxide, and 50 g of water were put in 1000 ml of ethylene glycol as an organic solvent, and then dissolved at 90 °C. The solution was refluxed at a high temperature of 190 °C. Upon completion of the reaction, a black precipitate was obtained. The precipitate was centrifuged by using a centrifugal separator at 4,000 rpm for 30 minutes, and then washed with ethanol and water for purification, thereby obtaining magnetic iron oxide particles with an average particle size of 200 nm.

### 2. Surface Introduction of Hydroxy Group (-OH)

5 g of the magnetic iron oxide particles were mixed with 200 ml of 1 M HCl, and then stirred using an ultrasonic dispersing unit for 10 minutes. The surface potential of the iron oxide particles before being mixed with HCl was not detected, while the surface potential of the iron oxide particles after being mixed with HCl was 33.6 mV. Therefore, it can be seen that as the surfaces of the iron oxide particles were etched, the surfaces of the particles were partially charged with positive charges.

Then, the etched iron oxide particles were mixed with an aqueous solvent to remove the remaining hydrochloric acid. Here, the positively charged iron oxide particles react with the water present in the aqueous solvent via a dehydrogenation reaction, and thus a plurality of hydroxy groups (-OH) were bonded to the surfaces of the particles. Here, the surface potential of the iron oxide particles after being mixed with the aqueous solvent was detected to be -23.5 mV.

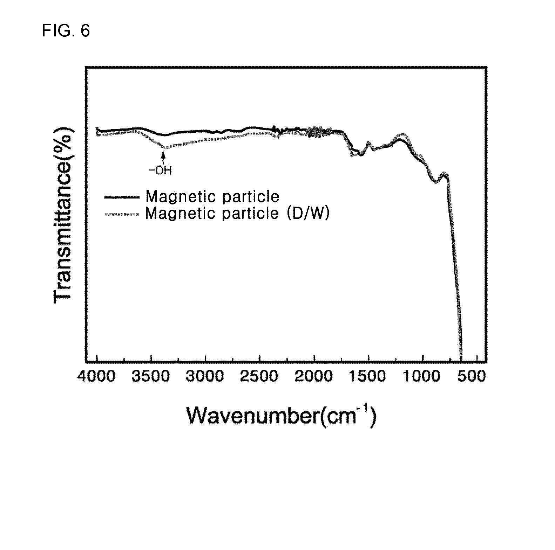

FIG. 6 is an image obtained by analyzing an iron oxide particle at an initial stage (in black) and an iron oxide particle after step (a-2) (in red) in Example 1, using fourier transform-infrared (FT-IR) spectroscopy. Referring to FIG. 6, it can be seen that after step (a-2), a peak was detected in the band of 3,400 cm⁻¹ to 3,650 cm⁻¹, which is typical of the hydroxy group. Therefore, it can be seen that the hydroxy group was introduced to the surfaces of the iron oxide particles.

### 3. Introduction of Organic Ligand

5 g of the hydroxy group-introduced iron oxide particles were mixed with 200 ml of oleic acid, and then dispersed for 30 minutes by using a dispersing unit, followed by stirring for 4 hours, thereby preparing the iron oxide particles surface-treated with oleic acid.

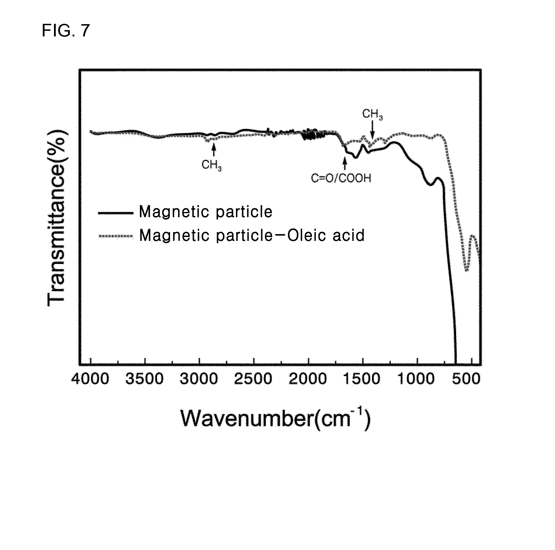

FIG. 7 is an image obtained by analyzing an iron oxide particle at an initial stage (in black) and an iron oxide particle after step (b) (in red) in Example 1, using FT-IR spectroscopy. Referring to FIG. 7, it can be seen that peaks were detected in the bands of 2,850 cm⁻¹ to 3,000 cm⁻¹ and 1,375 cm⁻¹ to 1,450 cm⁻¹, which are typical of the alkyl group being a constituent molecule of the oleic acid. Further, it can be seen that a peak was detected in the band of 1710 cm⁻¹, which is typical of the carboxyl group being a constituent molecule of the oleic acid. Therefore, it can be seen that the oleic acid was introduced onto the surfaces of the magnetic particles.

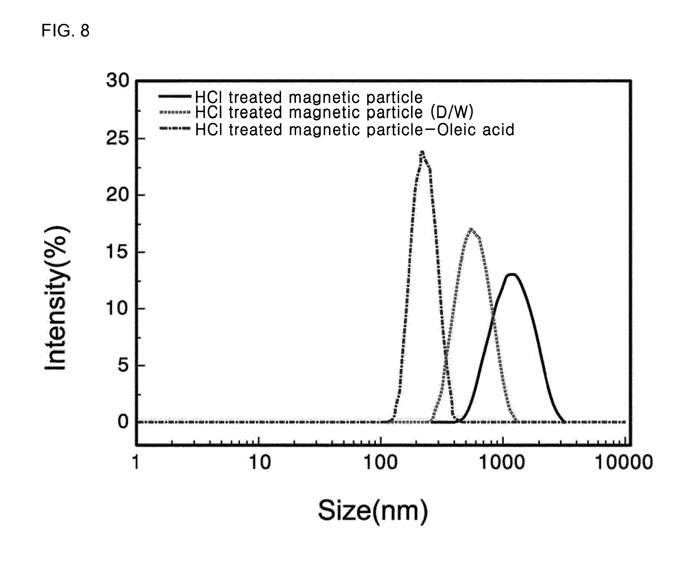

FIG. 8 shows data obtained by measuring the degree of dispersion of the particles as step (a-1) (in black), step (a-2) (in red), and step (b) (in blue) are carried out in Example 1.

Referring to FIG. 8, since the iron oxide particles should not agglomerate together in order to improve the degree of dispersion thereof, they need to have a size close to 200 nm, which is that of the initial iron oxide particles. In addition, when the degree of dispersion of the iron oxide particles is improved, the intensity of the uniform particles should have a large value. It can be seen that as the particle surface treatment steps according to the present invention were respectively conducted, the size of the iron oxide particles gradually approached 200 nm, which is the size of the initial iron oxide particles, and the intensity of the particles having the size of the initial iron oxide particles was gradually increased. It can be seen from these results that the dispersibility of the particles was improved as the particle surface treatment steps were respectively conducted.

### Example 2

### 1. Preparation of Magnetic Iron Oxide Particles

Magnetic iron oxide particles were obtained in the same manner as the preparation of magnetic iron oxide particles in Example 1.

### 2. Surface introduction of hydroxy group (-OH)

Hydroxy group-introduced magnetic iron oxide particles were obtained in the same manner as the surface introduction of hydroxy group (-OH) in Example 1.

### 3. Introduction of Organic Ligand

10 g of octadecyl amine (5 wt% solid content) was mixed in 190 g of tetrachloroethylene as a non-polar solvent to prepare 200 g of an organic ligand precursor.

5 g of the hydroxy group-introduced iron oxide particles were mixed with the organic ligand precursor, and then dispersed by using a dispersing unit for 30 minutes, followed by stirring for 4 hours to prepare the iron oxide particles surface-treated with octadecyl amine.

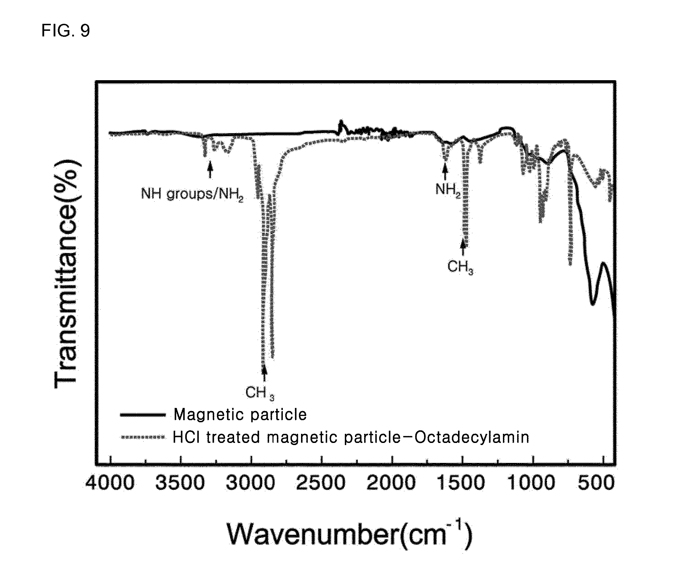

FIG. 9 is an image obtained by analyzing an iron oxide particle at an initial stage (in black) and an iron oxide particle after the introduction of an organic ligand (in red) in Example 2, using FT-IR spectroscopy. Referring to FIG. 9, it can be seen that peaks were detected in the bands of 2,850 cm⁻¹ to 3,000 cm⁻¹ and 1,375 cm⁻¹ to 1,450 cm⁻¹, which are typical of the alkyl group being a constituent molecule of the octadecyl amine. In addition, it can be seen that peaks were detected in the bands of 3,300 cm⁻¹ to 3,500 and 1600 cm⁻¹, which are typical of the amine group being a constituent molecule of the octadecyl amine. Therefore, it can be seen that the octadecyl amine was introduced onto the surfaces of the magnetic particles.

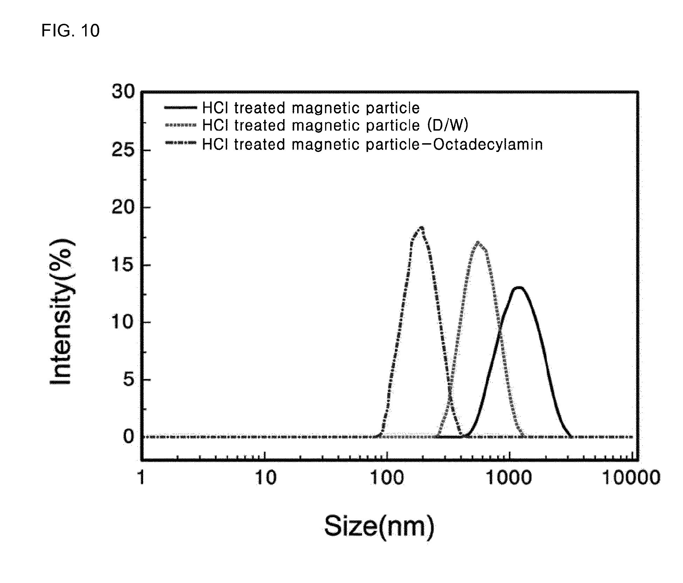

FIG. 10 shows data obtained by measuring the degree of dispersion of the particles prepared in Example 2.

Referring to FIG. 10, the degree of dispersion of the particles as step (a-1), step (a-2), and step (b) were carried out are indicated in black, red, and blue, respectively.

It can be seen that as the particle surface treatment steps according to the present invention were respectively conducted, the size of the iron oxide particles gradually approached 200 nm, which is the size of the initial iron oxide particles, and the intensity of the particles having the size of the initial iron oxide particles was gradually increased. It can be seen from these results that the dispersibility of the particles was improved as the particle surface treatment steps were respectively conducted.

### Example 3

### 1. Preparation of Magnetic Iron Oxide Particles

Magnetic iron oxide particles were obtained in the same manner as the preparation of magnetic iron oxide particles in Example 1.

### 2. Surface Introduction of Hydroxy Group (-OH)

Hydroxy group-introduced magnetic iron oxide particles were obtained in the same manner as the surface introduction of hydroxy group (-OH) in Example 1.

### 3. Introduction of Organic Ligand

20g of polystyrene (10 wt% solid content) was mixed in 180g of tetrachloroethylene as a non-polar solvent to prepare 200 g of an organic ligand precursor.

5 g of the hydroxy group-introduced iron oxide particles were mixed with the organic ligand precursor, and then dispersed by using a dispersing unit for 30 minutes, followed by stirring for 4 hours to prepare the iron oxide particles surface-treated with polystyrene.

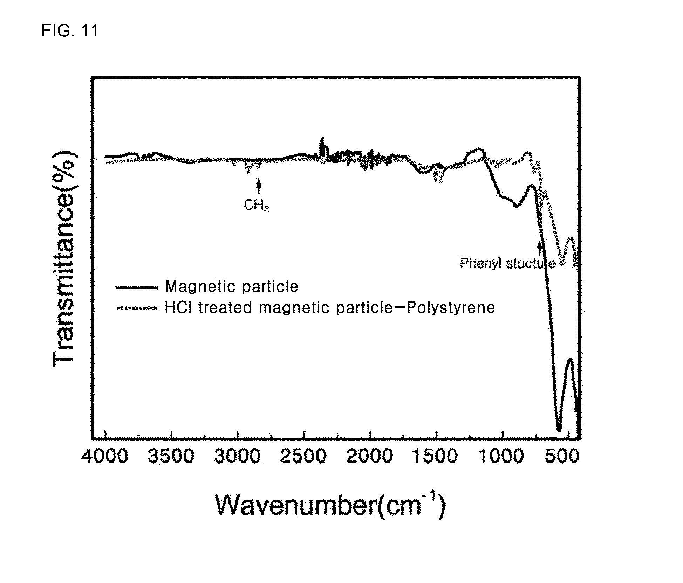

FIG. 11 is an image obtained by analyzing an iron oxide particle at an initial stage (in black) and an iron oxide particle after the introduction of an organic ligand (in red) in Example 3, using FT-IR spectroscopy. Referring to FIG. 11, it can be seen that a peak was detected in the band of 2,850 cm⁻¹ to 3,000 cm⁻¹, which is typical of the alkyl group constituting the polystyrene. Further, it can be seen that a peak was detected in the band of 700 cm⁻¹ to 900 cm⁻¹, which is typical of the phenyl group constituting the polystyrene. Therefore, it can be seen that the polystyrene was introduced onto the surfaces of the magnetic particles.

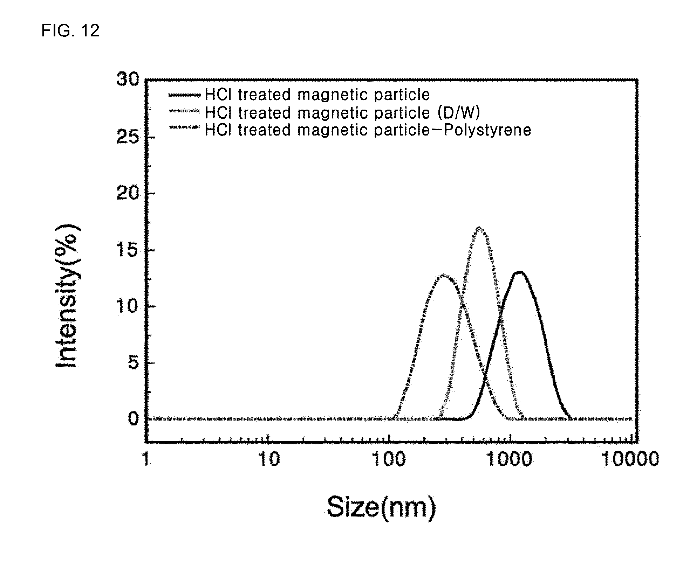

FIG. 12 shows data obtained by measuring the degree of dispersion of the particles prepared in Example 3.

Referring to FIG. 12, the degree of dispersion of the particles as step (a-1), step (a-2), and step (b) were carried out are indicated in black, red, and blue, respectively.

It can be seen that as the particle surface treatment steps according to the present invention were respectively conducted, the size of the iron oxide particles gradually approached 200 nm, which is the size of the initial iron oxide particles, and the intensity of the particles having the size of the initial iron oxide particles was gradually increased. It can be seen from these results that the dispersibility of the particles was improved as the particle surface treatment steps were respectively conducted.

### Example 4

### 1. Preparation of Magnetic Iron Oxide Particles

Magnetic iron oxide particles were obtained in the same manner as the preparation of magnetic iron oxide particles in Example 1.

### 2. Surface Introduction of Hydroxy Group (-OH)

Hydroxy group-introduced magnetic iron oxide particles were obtained in the same manner as the surface introduction of hydroxy group (-OH) in Example 1.

### 3. Introduction of Organic Ligand

10g of monostearin (5 wt% solid content) was mixed in 190g of tetrachloroethylene as a non-polar solvent to prepare 200 g of an organic ligand precursor.

5 g of the hydroxy group-introduced iron oxide particles were mixed with the organic ligand precursor, and then dispersed by using a dispersing unit for 30 minutes, followed by stirring for 4 hours to prepare the iron oxide particles surface-treated with monostearin.

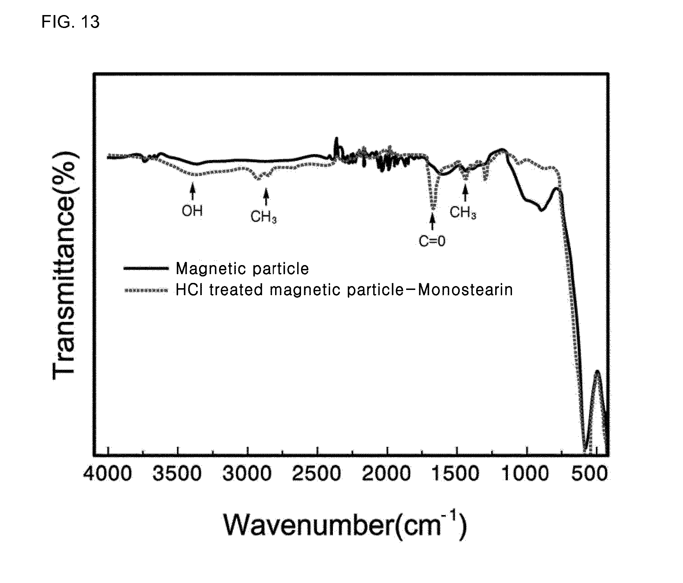

FIG. 13 is an image obtained by analyzing an iron oxide particle at an initial stage (in black) and an iron oxide particle after introduction of an organic ligand (in red) in Example 4, using FT-IR spectroscopy. Referring to FIG. 13, it can be seen that peaks were detected in the bands of 2,850 cm⁻¹ to 3,000 cm⁻¹ and 1,375 cm⁻¹ to 1,450 cm⁻¹, which are typical of the alkyl group being a constituent molecule of the monostearin. Further, it can be seen that a peak was detected in the band of 1,670 cm⁻¹ to 1,780 cm⁻¹, which is typical of the carbonyl group being a constituent molecule of the monostearin. Therefore, it can be seen that the monostearin was introduced onto the surfaces of the magnetic particles.

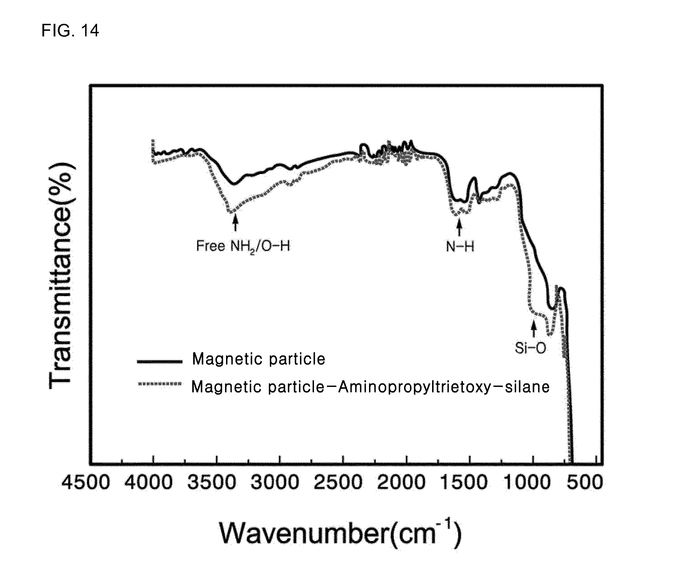

FIG. 14 shows data obtained by measuring the degree of dispersion of the particles prepared in Example 4.

Referring to FIG. 14, the degree of dispersion of the particles as step (a-1), step (a-2), and step (b) were carried out are indicated in black, red, and blue, respectively.

It can be seen that as the particle surface treatment steps according to the present invention were respectively conducted, the size of the iron oxide particles gradually approached 200 nm, which is the size of the initial iron oxide particles, and the intensity of the particles having the size of the initial iron oxide particles was gradually increased. It can be seen from these results that the dispersibility of the particles was improved as the particle surface treatment steps were respectively conducted.

### Example 5

### 1. Preparation of Surface Treatment Agent

3 ml of aminopropyltriethoxy-silane (APS) was mixed in 120 ml of water. The mixture was stirred by using a stirrer at 250 rpm for 1 hour to prepare a surface treatment agent.

### 2. Preparation of Magnetic Iron Oxide Particles

Magnetic iron oxide particles were obtained in the same manner as the preparation of magnetic iron oxide particles in Example 1.

### 3. Surface Introduction of Hydroxy Group (-OH)

Hydroxy group-introduced magnetic iron oxide particles were obtained in the same manner as the surface introduction of hydroxy group (-OH) in Example 1.

### 4. Introduction of Silicon Oxide Containing Amine Group

The hydroxy group-introduced magnetic iron oxide particles were mixed with the prepared surface treatment agent. Then, the mixture was heated at 30 °C for 2 hours, then heated for 9 minutes after the temperature was raised to 60 °C, and heated again at 30 °C for 2 hours. Then, the resultant mixture was washed three times with ethanol and then washed three times with purified water, thereby preparing a magnetic composite in which silicon oxide and an amine group were introduced onto the surface of the magnetic particle.

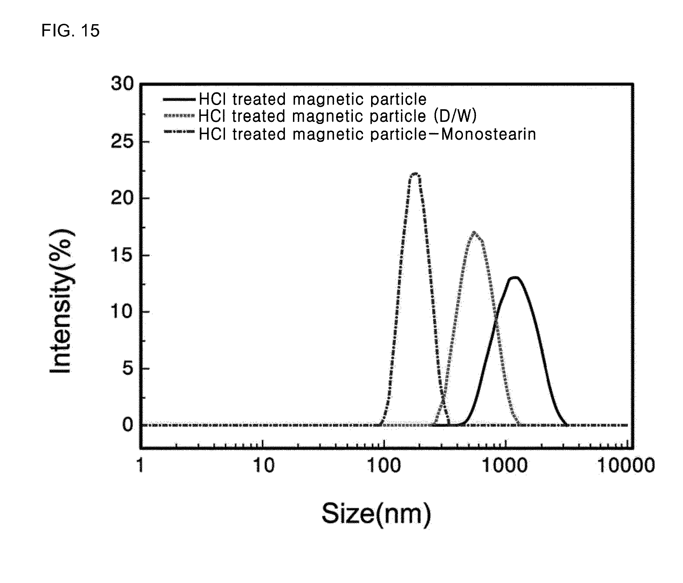

FIG. 15 is an image obtained by analyzing an iron oxide particle to which silicon oxide and an amine group are introduced after a hydroxy group has been introduced thereto in Example 5, using FT-IR spectroscopy.

Referring to FIG. 15, it can be seen that peaks were detected in the bands of 3,300 cm⁻¹ to 3,500 cm⁻¹ and 1,500 cm⁻¹, which are typical of the amine group. Here, it appears that the detected peaks of the amine group overlap with those of the hydroxy group (3,400 cm⁻¹ to 3,650 cm⁻¹). Further, it can be seen that a peak was detected in the band of 1,050 cm⁻¹ to 1,300 cm⁻¹ of the silicon oxide. Therefore, it can be seen that the silicon oxide and the amine group were introduced onto the surface of the magnetic particle.

### Example 6

### 1. Preparation of Surface Treatment Agent

1 ml of aminopropyltriethoxy-silane (APS) and 1 ml of tetraethylorthosilicate (TEOS) were mixed in 120 ml of water. The mixture was stirred by using a stirrer at 250 rpm for 1 hour to prepare a surface treatment agent.

### 2. Preparation of Magnetic Iron Oxide Particles

Magnetic iron oxide particles were obtained in the same manner as the preparation of magnetic iron oxide particles in Example 1.

### 3. Surface Introduction of Hydroxy Group (-OH)

Hydroxy group-introduced magnetic iron oxide particles were obtained in the same manner as the surface introduction of hydroxy group (-OH) in Example 1.

### 4. Introduction of Silicon Oxide Containing Amine Group

The hydroxy group-introduced magnetic iron oxide particles were mixed with the prepared surface treatment agent. Then, the mixture was heated at 60 °C for 24 hours, and then heated at 30 °C for 2 hours. Then, the resultant mixture was washed three times with ethanol and then washed three times with purified water, thereby preparing a magnetic composite in which silicon oxide and an amine group were introduced onto the surface of the magnetic particle.

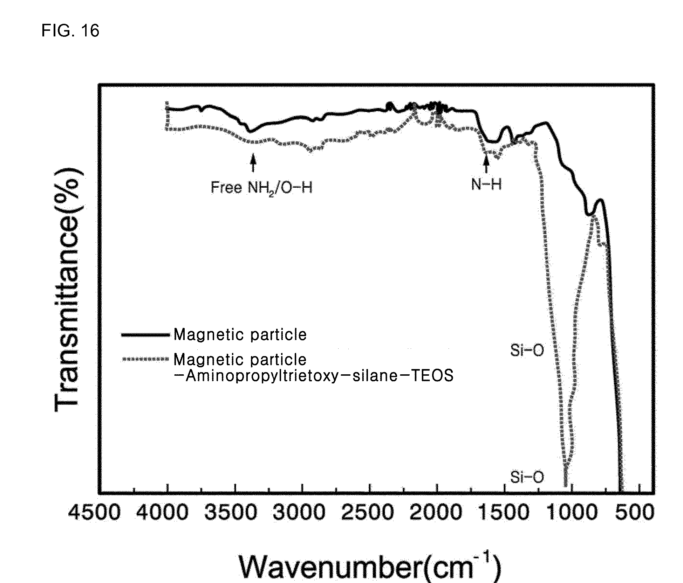

FIG. 16 is an image obtained by analyzing an iron oxide particle to which silicon oxide and an amine group are introduced after a hydroxy group has been introduced thereto in Example 6, using FT-IR spectroscopy.

Referring to FIG. 16, it can be seen that peaks were detected in the bands of 3,300 cm⁻¹ to 3,500 cm⁻¹ and 1,500 cm⁻¹, which are typical of the amine group. Here, it appears that the detected peaks of the amine group overlap with those of the hydroxy group (3,400 cm⁻¹ to 3,650 cm⁻¹). Further, it can be seen that a peak was detected in the band of 1,050cm⁻¹ to 1,300 cm⁻¹ of the silicon oxide. Therefore, it can be seen that the silicon oxide and the amine group were introduced onto the surface of the magnetic particle.

### Example 7

### 1. Preparation of Surface Treatment Agent

3 ml of (2-aminoethyl)-3-aminopropyl trimethylsilane was mixed in 120 ml of water. The mixture was stirred by using a stirrer at 250 rpm for 1 hour to prepare a surface treatment agent.

### 2. Preparation of Magnetic Iron Oxide Particles

Magnetic iron oxide particles were obtained in the same manner as the preparation of magnetic iron oxide particles in Example 1.

### 3. Surface Introduction of Hydroxy Group (-OH)

Hydroxy group-introduced magnetic iron oxide particles were obtained in the same manner as the surface introduction of hydroxy group (-OH) in Example 1.

### 4. Introduction of Silicon Oxide Containing Amine Group

The hydroxy group-introduced magnetic iron oxide particles were mixed with the prepared surface treatment agent. After that, the mixture was heated at 30 °C for 2 hours, and then heated for 9 minutes after the temperature was raised to 60 °C. Then, the resultant mixture was washed three times with ethanol and then washed three times with purified water, thereby preparing a magnetic composite in which silicon oxide and an amine group were introduced onto the surface of the magnetic particle.

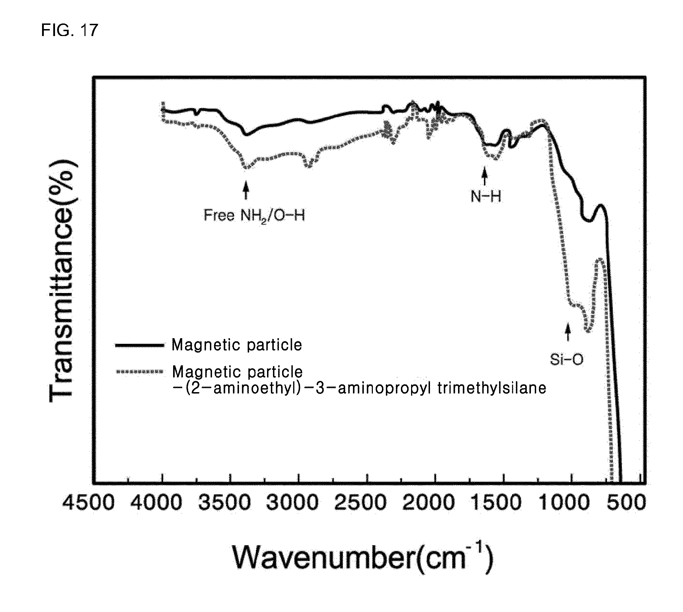

FIG. 17 is an image obtained by analyzing an iron oxide particle to which silicon oxide and an amine group are introduced after a hydroxy group has been introduced thereto in Example 7, using FT-IR spectroscopy.

Referring to FIG. 17, it can be seen that peaks were detected in the bands of 3,300 cm⁻¹ to 3,500 cm⁻¹ and 1,500 cm⁻¹, which are typical of the amine group. Here, it appears that the detected peaks of the amine group overlap with those of the hydroxy group (3,400 cm⁻¹ to 3,650 cm⁻¹). Further, it can be seen that a peak was detected in the band of 1,050 cm⁻¹ to 1,300 cm⁻¹ of the silicon oxide. Therefore, it can be seen that the silicon oxide and the amine group were introduced onto the surface of the magnetic particle.

### Example 8

### 1. Preparation of Surface Treatment Agent

10 ml of 5 wt% polyacrylic acid was mixed in 120 ml of water. The mixture was stirred by using a stirrer at 250 rpm for 1 hour to prepare a surface treatment agent.

### 2. Preparation of Magnetic Iron Oxide Particles

Magnetic iron oxide particles were obtained in the same manner as the preparation of magnetic iron oxide particles in Example 1.

### 3. Surface Introduction of Hydroxy Group (-OH)

Hydroxy group-introduced magnetic iron oxide particles were obtained in the same manner as the surface introduction of hydroxy group (-OH) in Example 1.

### 4. Introduction of Carboxyl Group

The hydroxy group-introduced magnetic iron oxide particles were mixed with the prepared surface treatment agent. Then, the mixture was heated at 30 °C for 3 hours, then heated for 10 minutes after the temperature was raised to 60 °C, and heated again at 30 °C for 1 hours. Then, the resultant mixture was washed three times with purified water, thereby preparing a magnetic composite in which a carboxyl group was introduced onto the surface of the magnetic particle.

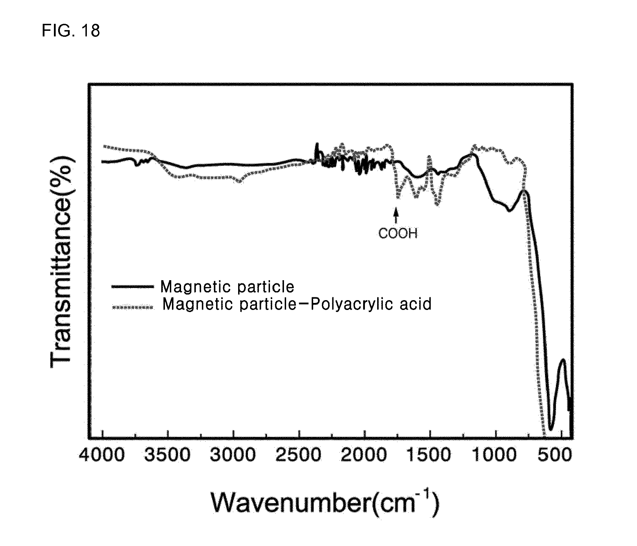

FIG. 18 is an image obtained by analyzing an iron oxide particle to which a carboxyl group is introduced after a hydroxy group has been introduced thereto in Example 8, using FT-IR spectroscopy.

Referring to FIG. 18, it can be seen that a peak was detected in the band of 1,670 cm⁻¹ to 1,780 cm⁻¹, which is typical of the carboxyl group. Therefore, it can be seen that the carboxyl group was introduced onto the surface of the magnetic particle.

### Example 9

### 1. Preparation of Surface Treatment Agent

2 g of methacrylic acid, 0.15 g of ethyleneglycol dimethacrylate and 0.15 g of azobisisobutyronitrile (AIBN) were mixed in 200 ml of ethanol. The mixture was stirred by using a stirrer at 250 rpm for 1 hour to prepare a surface treatment agent.

### 2. Preparation of Magnetic Iron Oxide Particle

Magnetic iron oxide particles were obtained in the same manner as the preparation of magnetic iron oxide particles in Example 1.

### 3. Surface Introduction of Hydroxy Group (-OH)

Hydroxy group-introduced magnetic iron oxide particles were obtained in the same manner as the surface introduction of hydroxy group (-OH) in Example 1.

### 4. Introduction of Carboxyl Group

The hydroxy group-introduced magnetic iron oxide particles were mixed with the prepared surface treatment agent. Then, the mixture was heated at 60 °C for 24 hours, and then heated at 30 °C for 2 hours. Then, the resultant mixture was washed three times with ethanol and then washed three times with purified water, thereby preparing a magnetic composite in which a carboxyl group was introduced onto the surface of the magnetic particle.

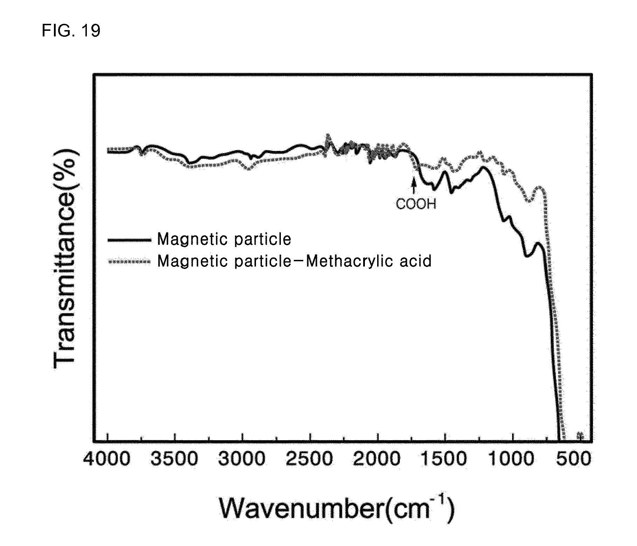

FIG. 19 is an image obtained by analyzing an iron oxide particle to which a carboxyl group is introduced after a hydroxy group has been introduced thereto in Example 9, using FT-IR spectroscopy.

Referring to FIG. 19, it can be seen that a peak was detected in the band of 1,670 cm⁻¹ to 1,780 cm⁻¹, which is typical of the carboxyl group. Therefore, it can be seen that the carboxyl group was introduced onto the surface of the magnetic particle.

### Example 10

### 1. Preparation of Surface Treatment Agent

20 ml of citric acid was mixed in 200 ml of water. The mixture was stirred by using a stirrer at 250 rpm for 1 hour to prepare a surface treatment agent.

### 2. Preparation of Magnetic Iron Oxide Particle

Magnetic iron oxide particles were obtained in the same manner as the preparation of magnetic iron oxide particles in Example 1.

### 3. Surface Introduction of Hydroxy Group (-OH)

5 g of the magnetic iron oxide particles were mixed with 200 ml of 1 M HCl, and then stirred using an ultrasonic dispersing unit for 30 minutes. The surface potential of the iron oxide particles before being mixed with HCl was not detected, while the surface potential of the iron oxide particles after being mixed with HCl was 33.6 mV. Therefore, it can be seen that as the surfaces of the iron oxide particles were etched, the surfaces of the particles were partially charged with positive charges.

Then, the etched iron oxide particles were mixed with an aqueous solvent to remove the remaining hydrochloric acid. Here, the positively charged iron oxide particles react with the water present in the aqueous solvent via a dehydrogenation reaction, and thus a plurality of hydroxy groups (-OH) were bonded to the surfaces of the particles. Here, the surface potential of the iron oxide particles after being mixed with the aqueous solvent was detected to be -23.5 mV.

### 4. Introduction of Carboxyl Group

The hydroxy group-introduced magnetic iron oxide particles were mixed with the prepared surface treatment agent. Then, the mixture was heated at 80 °C for 12 hours, and then heated at 30 °C for 1 hour. Then, the resultant mixture was washed three times with ethanol and then washed three times with purified water, thereby preparing a magnetic composite in which a carboxyl group was introduced onto the surface of the magnetic particle.

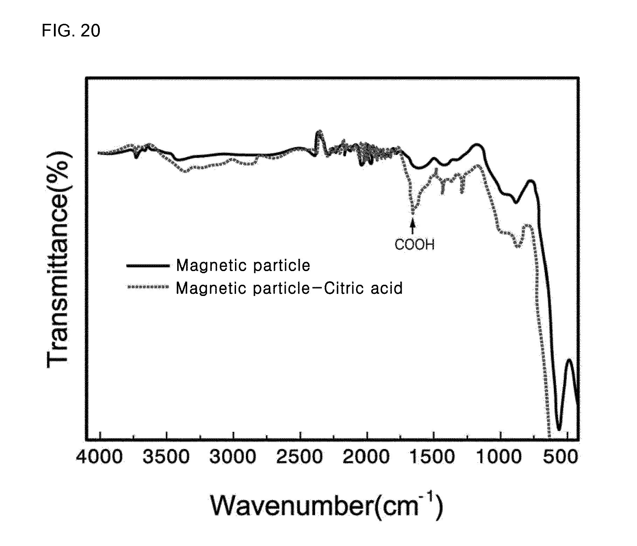

FIG. 20 is an image obtained by analyzing an iron oxide particle to which a carboxyl group is introduced after a hydroxy group has been introduced thereto in Example 10, using FT-IR spectroscopy.

Referring to FIG. 20, it can be seen that a peak was detected in the band of 1,670 cm⁻¹ to 1,780 cm⁻¹, which is typical of the carboxyl group. Therefore, it can be seen that the carboxyl group was introduced onto the surface of the magnetic particle.

## Claims

1. A surface treatment method for a magnetic particle, comprising the steps of:
(a) treating a magnetic particle with acid and then mixing the acid-treated magnetic particle with a water-soluble solvent to form a hydroxy group (-OH) on a surface of the magnetic particle; and
(b) mixing the magnetic particle on which the hydroxy group is formed with a surface treatment agent containing an organic ligand capable of bonding to the hydroxy group to introduce the organic ligand onto the surface of the magnetic particle.

2. The method of claim 1, wherein in step (a), the magnetic particle includes at least one of Cr, Ni, Ti, Zr, Fe, Co, Zn, Gd, Ta, Nb, Pt, Au, Mg, Mn, Pd, Sr, Ag, Ba, Cu, W, Mo, Sn, and Pb.

3. The method of claim 1, wherein in step (a), the acid includes at least one of hydrochloric acid (HCl), acetic acid (CH₃COOH), sulfuric acid (H₂SO₄), nitric acid (HNO₃), formic acid, citric acid, lactic acid, and amino acid.

4. The method of claim 1, wherein in step (b), the surface treatment agent containing the organic ligand includes at least one of a carboxyl group, a hydroxy group, an amine group, a vinyl group, an acrylate group, an alcohol group, a ketone group, an ester group, and an aldehyde group.

5. The method of claim 1, wherein in step (b), the surface treatment containing the organic ligand includes at least one of ricinoleic acid, linoleic acid, monostearin, palmitic acid, octadecylamin, trioctylphosphine oxide, oleic acid, stearic acid, polymethylmethacrylate, polystyrene, solbitol monooleate, sorbitan trioleate, myristoleic acid, palmitoleic acid, sapienic acid, arachidonic acid, α-linolenic acid, eicosapentaenoic acid, erucic acid, docosahexaenoic acid, trioctylphosphate, hexadecylamino, fatty acid series, and olefin series.

6. A magnetic composite being prepared by:
(a) treating a magnetic particle with acid and then mixing the acid-treated magnetic particle with a water-soluble solvent to form a hydroxy group (-OH) on a surface of the magnetic particle; and
(b) mixing the magnetic particle on which the hydroxy group is formed with a surface treatment agent containing an organic ligand capable of bonding to the hydroxy group to introduce the organic ligand onto the surface of the magnetic particle.

7. The magnetic composite of claim 6, wherein the magnetic particle is a cluster resulting from agglomeration of a plurality of magnetic particles.

8. The magnetic composite of claim 7, wherein, when the cluster is dispersed in a water-soluble or fat-soluble solvent, a steric hindrance effect is generated among the plurality of magnetic particles by the organic ligand formed on the surfaces of the plurality of magnetic particles.

9. A surface treatment method for a magnetic particle, comprising the steps of:
(a) mixing a surface precursor with a solvent to prepare a surface treatment agent, the surface precursor containing silicon substituted with at least one alkoxy group and at least one amine group;
(b) treating a magnetic particle with acid and then mixing the acid-treated magnetic particle with a water-soluble solvent to form a hydroxy group (-OH) on a surface of the magnetic particle; and
(c) mixing the magnetic particle on which the hydroxy group is formed with the surface treatment agent to introduce silicon oxide containing the amine group onto the surface of the magnetic particle.

10. The method of claim 9, wherein in step (a), the amine group is selected from a group consisting of a monoamine group, a diamine group, a triamine group, an ethylene diamine group, and a diethylene triamine group.

11. The method of claim 9, wherein, in step (a), the surface precursor includes aminopropyltriethoxy-silane.

12. The method of claim 9, wherein, in step (a), the solvent includes at least one of water and a hydrophilic solvent.

13. The method of claim 9, wherein in step (b), the magnetic particle includes at least one of Cr, Ni, Ti, Zr, Fe, Co, Zn, Gd, Ta, Nb, Pt, Au, Mg, Mn, Pd, Sr, Ag, Ba, Cu, W, Mo, Sn, and Pb.

14. The method of claim 9, wherein in step (b), the acid includes at least one of hydrochloric acid (HCl), acetic acid (CH₃COOH), sulfuric acid (H₂SO₄), nitric acid (HNO₃), formic acid, citric acid, lactic acid, and amino acid.

15. The method of claim 9, wherein step (c) is conducted at a temperature ranging from 25 °C to 90 °C.

16. The method of claim 9, wherein step (c) is conducted in a combination of a first temperature maintenance state at 25 °C to 35 °C and a second temperature maintenance state at 60 °C to 90 °C.

17. The method of claim 16, wherein heating in the first temperature maintenance state, heating in the second temperature maintenance state, and heating again in the first temperature maintenance state are sequentially conducted.

18. A magnetic composite being prepared by:
(a) mixing a surface precursor with a solvent to prepare a surface treatment agent, the surface precursor containing silicon substituted with at least one alkoxy group and at least one amine group;
(b) treating a magnetic particle with acid and then mixing the acid-treated magnetic particle with a water-soluble solvent to form a hydroxy group (-OH) on a surface of the magnetic particle; and
(c) mixing the magnetic particle on which the hydroxy group is formed with the surface treatment agent to introduce silicon oxide containing the amine group onto the surface of the magnetic particle.

19. A magnetic composite for labeling a target material, the magnetic composite being prepared by:
(a) mixing a surface precursor with a solvent to prepare a surface treatment agent, the surface precursor containing silicon substituted with at least one alkoxy group and at least one amine group;
(b) treating a magnetic particle with acid and then mixing the acid-treated magnetic particle with a water-soluble solvent to form a hydroxy group (-OH) on a surface of the magnetic particle; and
(c) mixing the magnetic particle on which the hydroxy group is formed with the surface treatment agent to introduce silicon oxide containing the amine group onto the surface of the magnetic particle,
wherein the amine group is directly or indirectly used to label a target material including at least one of DNA, RNA, peptide, protein, antigen, antibody, nucleic acid aptamer, hapten, antigen protein, DNA-binding protein, hormone, tumor-specific marker, and tissue-specific marker.

20. A surface treatment method for a magnetic particle, comprising the steps of:
(a) mixing a surface precursor with a solvent to prepare a surface treatment agent, the surface precursor containing at least one carboxyl group and at least one other functional group capable of undergoing a dehydration reaction with a hydroxy group (-OH);
(b) treating a magnetic particle with acid and then mixing the acid-treated magnetic particle with a water-soluble solvent to form a hydroxy group (-OH) on a surface of the magnetic particle; and
(c) mixing the magnetic particle on which the hydroxy group is formed with the surface treatment agent to introduce the carboxyl group onto the surface of the magnetic particle through a dehydration reaction of the other functional group with the hydroxy group.

21. A surface treatment method for a magnetic particle, comprising the steps of:
(a) mixing a surface precursor with a solvent to prepare a surface treatment agent, the surface precursor containing at least one first carboxyl group and at least one second carboxyl group;
(b) treating a magnetic particle with acid and then mixing the acid-treated magnetic particle with a water-soluble solvent to form a hydroxy group (-OH) on a surface of the magnetic particle; and
(c) mixing the magnetic particle on which the hydroxy group is formed with the surface treatment agent to introduce the first carboxyl group onto the surface of the magnetic particle through a dehydration reaction of the second carboxyl group with the hydroxy group without a dehydration reaction of the first carboxyl group with the hydroxy group.

22. The method of claim 20, wherein, in step (a), the surface precursor includes hydroxy acid based compounds.

23. The method of claim 21, wherein, in step (a), the surface precursor includes acrylic acid based compounds.

24. The method of claim 20 or 21, wherein, in step (a), the solvent includes at least one of water and a hydrophilic solvent.

25. The method of claim 20 or 21, wherein in step (b), the magnetic particle includes at least one of Cr, Ni, Ti, Zr, Fe, Co, Zn, Gd, Ta, Nb, Pt, Au, Mg, Mn, Pd, Sr, Ag, Ba, Cu, W, Mo, Sn, and Pb.

26. The method of claim 20 or 21, wherein in step (b), the acid includes at least one of hydrochloric acid (HCl), acetic acid (CH₃COOH), sulfuric acid (H₂SO₄), nitric acid (HNO₃), formic acid, citric acid, lactic acid, and amino acid.

27. The method of claim 20 or 21, wherein step (c) is conducted at a temperature ranging from 25 °C to 90 °C.

28. The method of claim 20 or 21, wherein step (c) is conducted in a combination of a first temperature maintenance state at 25 °C to 35 °C and a second temperature maintenance state at 60 °C to 90 °C.

29. The method of claim 28, wherein heating in the first temperature maintenance state, heating in the second temperature maintenance state, and heating again in the first temperature maintenance state are sequentially conducted.

30. A magnetic composite being prepared by:
(a) mixing a surface precursor with a solvent to prepare a surface treatment agent, the surface precursor containing at least one carboxyl group and at least one other functional group capable of undergoing a dehydration reaction with a hydroxy group (-OH);
(b) treating a magnetic particle with acid and then mixing the acid-treated magnetic particle with a water-soluble solvent to form a hydroxy group (-OH) on a surface of the magnetic particle; and
(c) mixing the magnetic particle on which the hydroxy group is formed with the surface treatment agent to introduce the carboxyl group onto the surface of the magnetic particle through a dehydration reaction of the other functional group with the hydroxy group.

31. A magnetic composite being prepared by:
(a) mixing a surface precursor with a solvent to prepare a surface treatment agent, the surface precursor containing at least one first carboxyl group and at least one second carboxyl group;
(b) treating a magnetic particle with acid and then mixing the acid-treated magnetic particle with a water-soluble solvent to form a hydroxy group (-OH) on a surface of the magnetic particle; and
(c) mixing the magnetic particle on which the hydroxy group is formed with the surface treatment agent to introduce the first carboxyl group onto the surface of the magnetic particle through a dehydration reaction of the second carboxyl group with the hydroxy group without a dehydration reaction of the first carboxyl group with the hydroxy group.

32. A magnetic composite for labeling a target material, the magnetic composite being prepared by:
(a) mixing a surface precursor with a solvent to prepare a surface treatment agent, the surface precursor containing at least one carboxyl group and at least one other functional group capable of undergoing a dehydration reaction with a hydroxy group (-OH);
(b) treating a magnetic particle with acid and then mixing the acid-treated magnetic particle with a water-soluble solvent to form a hydroxy group (-OH) on a surface of the magnetic particle; and
(c) mixing the magnetic particle on which the hydroxy group is formed with the surface treatment agent to introduce the carboxyl group onto the surface of the magnetic particle through a dehydration reaction of the other functional group with the hydroxy group,
wherein the carboxyl group is directly or indirectly used to label a target material including at least one of DNA, RNA, peptide, protein, antigen, antibody, nucleic acid aptamer, hapten, antigen protein, DNA-binding protein, hormone, tumor-specific marker, and tissue-specific marker.

33. A magnetic composite for labeling a target material, the magnetic composite being prepared by:
(a) mixing a surface precursor with a solvent to prepare a surface treatment agent, the surface precursor containing at least one first carboxyl group and at least one second carboxyl group;
(b) treating a magnetic particle with acid and then mixing the acid-treated magnetic particle with a water-soluble solvent to form a hydroxy group (-OH) on a surface of the magnetic particle; and
(c) mixing the magnetic particle on which the hydroxy group is formed with the surface treatment agent to introduce the first carboxyl group onto the surface of the magnetic particle through a dehydration reaction of the second carboxyl group with the hydroxy group without a dehydration reaction of the first carboxyl group with the hydroxy group,
wherein the first carboxyl group is directly or indirectly used to label a target material including at least one of DNA, RNA, peptide, protein, antigen, antibody, nucleic acid aptamer, hapten, antigen protein, DNA-binding protein, hormone, tumor-specific marker, and tissue-specific marker.
